# EUROPEAN PATENT SPECIFICATION

(11) **EP 3 853 256 B1**
(45) Date of publication and mention of the grant of the patent: **21.08.2024**
(21) Application number: 19831921.2
(22) Date of filing: 19.09.2019
(51) Int. Cl.: C07K 16/28, A61K 39/395, A61P 35/04

(54) **DUAL ACTING CD1D IMMUNOGLOBULIN**
ZWEIFACH WIRKENDES CD1D-IMMUNOGLOBULIN
IMMUNOGLOBULINE CD1D À DOUBLE ACTION

(30) Priority: 19.09.2018 NL 2021664
(43) Date of publication of application: 28.07.2021
(73) Proprietor: LAVA Therapeutics N.V., 3584 CM Utrecht (NL)
(72) Inventor: VAN DER VLIET, Johannes Jelle, 3584 CM Utrecht (NL); LAMERIS, Roeland, 3584 CM Utrecht (NL); DE GRUIJL, Tanja Denise, 3584 CM Utrecht (NL); PARREN, Paul Willem Henri Ida, 3584 CM Utrecht (NL)
(74) Representative: Cooley (UK) LLP
(86) International application number: PCT/NL2019/050624
(87) International publication number: WO 2020/060405

(56) References cited:
- WO-A1-2013/053021
- WO-A1-2015/156673
- WO-A1-2016/001276
- WO-A1-2016/122320
- BACHY EMMANUEL ET AL: "CD1d-restricted peripheral T cell lymphoma in mice and humans.", THE JOURNAL OF EXPERIMENTAL MEDICINE 02 05 2016, vol. 213, no. 5, 2 May 2016 (2016-05-02), pages 841 - 857, XP002791465, ISSN: 1540-9538
- NAMBIAR JONATHAN ET AL: "Potent neutralizing anti-CD1d antibody reduces lung cytokine release in primate asthma model", MABS, vol. 7, no. 3, May 2015 (2015-05-01), pages 638 - 650, XP002791466
- SPINOZZI F ET AL: "Local expansion of allergen-specific CD30+Th2-type gamma delta T cells in bronchial asthma.", MOLECULAR MEDICINE (CAMBRIDGE, MASS.) NOV 1995, vol. 1, no. 7, November 1995 (1995-11-01), pages 821 - 826, XP002791467, ISSN: 1076-1551
- MIGALOVICH SHEIKHET HELENA ET AL: "Dysregulated CD25 and Cytokine Expression by [gamma][delta] T Cells of Systemic Sclerosis Patients Stimulated With Cardiolipin and Zoledronate.", FRONTIERS IN IMMUNOLOGY 2018, vol. 9, 2018, pages 753, XP002791468, ISSN: 1664-3224
- LAMERIS ROELAND ET AL: "Generation and characterization of CD1d-specific single-domain antibodies with distinct functional features.", IMMUNOLOGY 09 2016, vol. 149, no. 1, September 2016 (2016-09-01), pages 111 - 121, XP002791464, ISSN: 1365-2567

## Description

### Field of the invention

The present invention relates to the field of immunology, more in particular to the field of binding molecules and/or immunoglobulins which bind to human CD1d, including antibodies and fragments thereof that modify CD1d-mediated biological functions such as activation and blocking of CD1d-restricted T-cells, including natural killer T (NKT) cells and gamma(y)-delta(δ) T-cells, and modulation of the function of cells expressing CD1d. The invention relates to bi-, tri- or multi-specific binding molecules and/or immunoglobulins that bind to CDld and a γδ T cell receptor (TCR) and/or a tumor target. The invention further relates to pharmaceutical preparations and use of such bi-, and tri-, or multi-specific binding molecules and/or immunoglobulins in the treatment of diseases or disorders.

### Background of the invention

CDld is a member of the CD1 (cluster of differentiation 1) family of glycoproteins (including CD1a, CD1b, CD1c, CDld and CD1e) and is expressed on the surface of various human cells, including antigen presenting cells (APC). They are non-classical MHC proteins, related to the class I MHC proteins, and are involved in the presentation of lipid antigens to a subgroup of T cells. In human COld is encoded by CD1D, also known as R3G1. APC displaying CDld include B-cells, dendritic cells (e.g. in lymph nodes), and monocytes. COld is also expressed by various other cell types, for example, in liver, pancreas, skin, kidney, uterus, conjunctiva, epididymis, thymus and tonsil (Canchis et al. (1992) Immunology 80:561).

Cells that are activated/stimulated via COld include CDld restricted Natural Killer T-cells (NKT cells). NKT cells are a heterogeneous group of T cells that share properties of both T cells and natural killer cells. NKT cells are a subset of T cells that express an alpha(α)/beta(β) T-cell receptor (TCR), as well a variety of molecular markers that are typically associated with NK cells.

Type 1 or invariant NKT (iNKT) cells are the best-studied group of NKT cells and differ from conventional αβ-T cells in that their T-cell receptors are far more limited in diversity ('invariant'). These invariant, but also other CD1d-restricted, NKT cells (type II NKT) recognize several antigens, such as (self or foreign) lipids, glycolipids, sulfatides, phospholipids, lipopeptides, hydrophobic peptides and/or amphipathic α-helical peptides, presented by CDld molecules present on APC (Enrico Girardi et al. (2016) J Biol Chem. 291(20): 10677). The interaction between (antigen-presenting) CDld and TCR triggers the release of cytokines including Th1- and/or Th2-like cytokines, such as interferon-y, tumor necrosis factor-a, and interleukins like IL-4, IL-5 and IL-13.

An α-galactosylceramide, KRN7000, is the best studied ligand of the lipid-binding COld in NKT cell activation in vitro and in vivo. Other ligands comprise isoglobotrihexosylceramide (for mouse iNKT cells), (microbial-derived) glycuronosylceramides, α-C-galactosylceramides, threitol ceramide, and a variety of (human and non-human) glycolipids such as lysophophatidylcholine and lysosphingomyelin (Fox et al (2009) PLOS Biology 7:10:e1000228).

Important roles of iNKT cells have been demonstrated in the regulation of autoimmune, allergic, antimicrobial, and antitumor immune responses (van der Vliet et al. (2004) Clin Immunol 112(1): 8). Physiologically, the NKT-cells can augment or inhibit immune responses, including antitumor, autoimmune, and anti-pathogen responses, through a variety of mechanisms depending on context (Yue et al. (2010) J Immunol 184: 268), including induction of cell death in multiple myeloma cells. Conditions in which (invariant) NKT-cells may be involved include autoimmune or inflammatory diseases, including myasthenia gravis, psoriasis, ulcerative colitis, primary biliary cirrhosis, colitis, autoimmune hepatitis, atherosclerosis, and asthma. In addition to cytokine release, NKT cell effector functions which result in cell lysis, such as perforin release and granzyme release and cell death, may also be relevant in conditions in which NKT cells are implicated, such as in cancer.

Based on their T cell receptor (TCR) usage and antigen specificities, CD1d-restricted NKT cells have been divided into two main subsets: type I NKT cells that use a canonical invariant TCR α-chain and recognize α-galactosylceramide (α-GalCer), and type II NKT cells that use a more diverse αβ-TCR repertoire and do not recognize α-GalCer. In addition, α-GalCer-reactive NKT cells that use non-canonical αβ-TCRs and CD1d-restricted T cells that use γδ- or δ/αβ-TCRs have recently been identified, revealing further diversity among CD1d-restricted T cells (Macho-Fernandez and Brigl (2015) Front Immunol 6:362).

### Type I NKT Cells

The discovery of the first COld-presented antigen, α-galactosylceramide (α-GalCer), by Kawano and colleagues in 1997 enabled several important steps forward in our understanding of NKT cell biology, particularly of type I or invariant NKT (iNKT) cells (Kawano et al. (1997) Science 278:1626). Type I NKT cells express an invariant Vα14Jα18 TCR α-chain in mice and Vα24Jα18 in humans, paired with a limited repertoire of TCR β-chains (Vβ8, Vβ7, Vβ2 in mice and exclusively Vβ11 in humans). Type I NKT cells can be highly autoreactive even at steady state and display an activated/memory phenotype with high surface levels of the activation markers CD69, CD44, and CD122 (IL-2R β-chain) and low expression of CD62L, a marker expressed by naïve T cells that home to lymph nodes (Bendelac et al (1992) J Exp Med 175:731 ; Matsuda et al. (2000) J Exp Med 192:741). Type I NKT cells critically contribute to natural anti-tumor responses, as demonstrated by the prompt growth of spontaneous tumors in type I NKT cell-deficient Jα18-/- mice compared to WT mice (Smyth et al. (2000) J Exp Med 191:661; Swann et al (2009) Blood 113:6382; Bellone et al (2010) PLoS One 5:e8646). Furthermore, the activation of type I NKT cells by α-GalCer provides potent effects against hematologic malignancies and solid tumors through their IFN-γ-production and the subsequent activation of dendritic cells (DC) and NK cells (Smyth et al (2002) Blood 99:1259; Berzofsky and Terabe (2008) J Immunol 180:3627). By contrast, sulfatide-activated type II NKT cells repress anti-tumor immunity (Terabe et al (2000) Nat Immunol 1:515; Terabe et al (2005) J Exp Med 202:1627; Renukaradhya et al (2008) Blood 111:5637) by abrogating type I NKT activation in response to α-GalCer, in terms of cytokine secretion and expansion (Ambrosino et al (2007) J Immunol 179:5126). Moreover, their IL-13 production, in combination with TNF-α, led to up-regulation of TGF-β secretion by myeloid-derived suppressor cells (MDSC), and resulted in decreased cytotoxic T cell activity (Terabe et al (2003) J Exp Med 198:1741).

### Type II NKT Cells

CD1d-restricted T cells that do not express the Vα14-Jα18 rearrangement and do not recognize α-GalCer were first described in MHC II-deficient mice among the remaining CD4+ T cells (Cardell Set al (1995) J Exp Med 182:993). From then called diverse NKT (dNKT), type II NKT, or variant NKT (vNKT) cells, this NKT cell population, found in both mice and humans, exhibits a more heterogeneous TCR repertoire. Several lines of evidence suggest that type II NKT cells can contribute to and modulate a range of immune responses, occasionally in opposing roles to type I NKT cells.

### yδ T cells

In addition to CD1d-restricted T cells with αβ TCRs, CD1d-restricted T cells expressing γδ TCRs have recently been described in both mice and humans. According to their Vδ-chain expression, human γδ T cells can be divided into two major populations: Vδ2+ and "non-Vδ2" subsets, the latter comprise, *inter alia,* Vδ1+γδ T cells and the less prevalent Vδ3+γδ T cells (McVay et al (1999) Crit Rev Immunol 19:431; Vantourout and Hayday (2013) Nat Rev Immunol 13:88). Vδ1+γδ T cells are mainly tissue resident and are found in the skin and at mucosal surfaces, whereas Vδ2+γδ T cells are predominant in human blood. Compared with αβ T cells, the types of antigens recognized by γδ T cells and the role and function of antigen presentation in γδ TCR recognition are much less clear. Interestingly, some γδ T cells have recently been found to directly recognize CD1d-presented lipid antigens (Hayday and Vantourout (2013) Immunity 39:994). In 2013, Uldrich et al (2013) Nature Immunol 14: 1137) had identified a γδ-T cell population that recognized COld in combination with select glycolipid antigens, including αGalCer. These T cells are referred to as CD1d-restricted Vδ1+ T cells. Without being bound to theory, it is believed that Vδ1+ T cells can exert an inflammatory response that can be counterproductive for an effective anti-tumor response.

### Summary of the invention

For the reasons indicated above, modulation of CD1d-mediated effects is of potential therapeutic benefit. There is an ongoing need for binding molecules that can specifically bind and/or interact with CD1d, both in vitro and in vivo. In particular there is need for such binding molecules that bind and/or modulate (activate or inhibit) biological functions that involve CDld such as, but not limited to, NKT-cell activation. Such binding molecules may, for example, show benefit in the various diseases in which CD1d-mediated functions play a role.

The inventors have previously identified a single domain antibody, designated 1D12 or VHH 12, that specifically activates iNKT (type I NKT) cells, without the need of CDld ligand (WO2016122320). The Examples of the present disclosure show for the first time that 1D12 is not only able to activate iNKT cells, but at the same time enables blocking of CD1d-restricted Vδ1+ T cell activation. This observation now leads to the notion that 1D12 can be used for the treatment of disorders caused, maintained and/or propagated by CD1d-restricted Vδ1+ T-cell activation. The skilled person will be aware that this specific use is not restricted to 1D12, but extends to any antibody that is functionally and/or structurally similar to 1D12.

In a first aspect, the invention provides a binding molecule comprising a first binding moiety of an antibody that is able to compete with 1D12 (SEQ ID NO: 4) in binding to a COld molecule and comprising a second binding moiety of an antibody that is able to specifically bind to a Vγ9Vδ2-TCR, wherein the binding molecule is able to activate iNKT cells and wherein the binding molecule is able to activate Vγ9Vδ2 T cells. The inventors have shown that a bispecific CD1d/Vγ9Vδ2-TCR antibody can induce degranulation of iNKT cells and Vγ9Vδ2-T cells and control growth of COld-expressing tumor cells. Furthermore, in a mouse multiple myeloma model, infusion of both iNKT cells and γδ T cells with a bispecific CD1d/Vγ9Vδ2-TCR antibody significantly prolonged survival compared to a mixture of the iNKT cells and γδ T cells alone without the antibody.

In a further aspect, the invention provides a composition according to the first aspect of the invention and a pharmaceutically acceptable excipient.

Further aspects and embodiments of the invention are described below.

### Brief description of the drawings

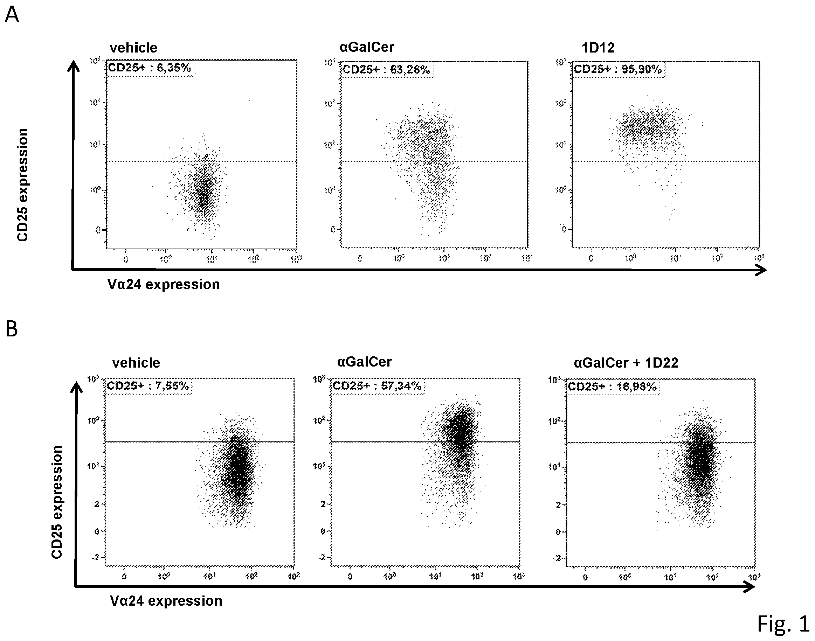

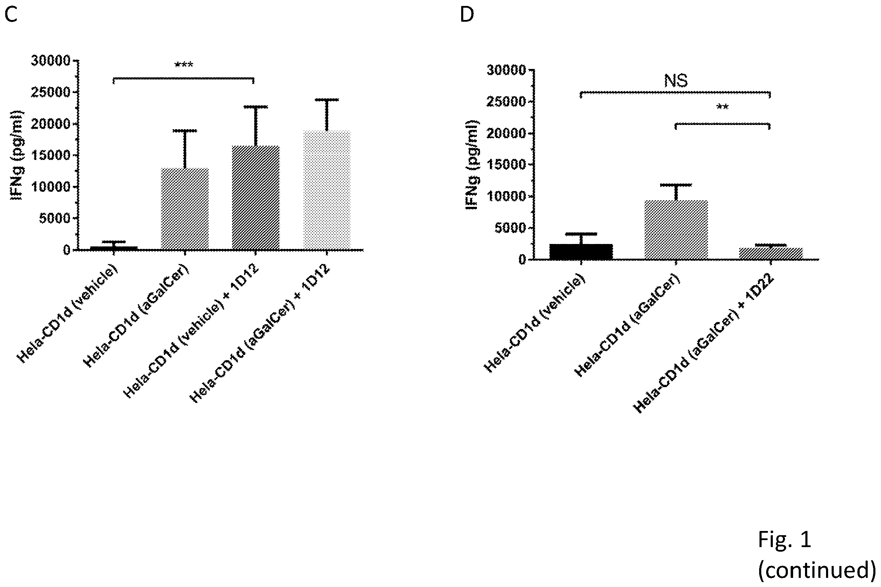

**Fig. 1****.** Anti-CD1d VHH mediated activation and inhibition of invariant natural killer T (iNKT) cells. iNKT CD25 expression and interferon-γ (IFN-y) production were determined after a 24-hr co-culture of iNKT cells with CD1d-transfected HeLa cells pulsed with vehicle control (vehicle) or αGalCer whether or not in combination with anti-CD1d VHH 1D12 (100 nM) or 1D22 (1000 nM). Representative dot-plots illustrating marked activation by 1D12 (A) or inhibition by 1D22 (B) of iNKT cells as depicted by CD25 expression, in addition to a marked increase (by 1D12) or decrease (by 1D22) in iNKT cell IFN-y production (C and D). Data represent mean + SD of 3-4 individual experiments with iNKT obtained from different donors, **P<0.01, ***P < 0.001, calculated with a one-way analysis of variance with Turkey's post hoc test.

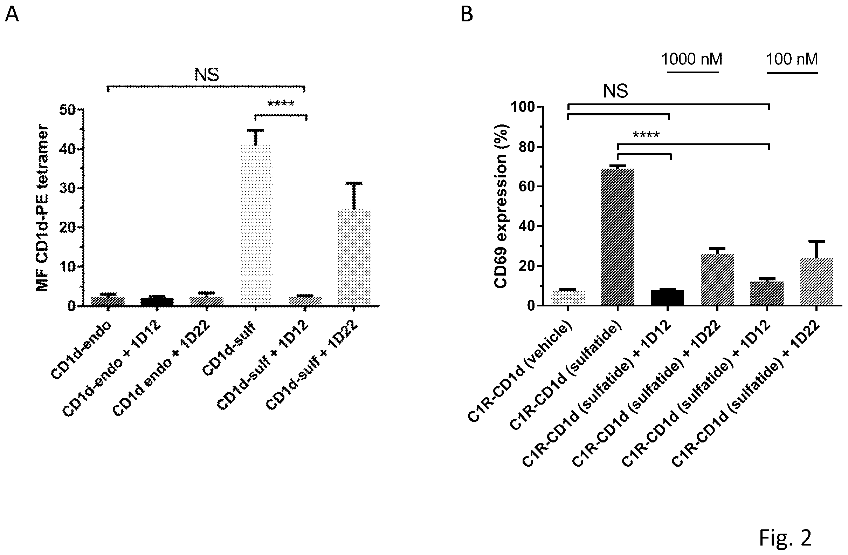

**Fig. 2****.** Vδ1-sulf-CD1d interaction is prevented by anti-CD1d VHH 1D12 and reduced by 1D22. Endogenous or sulfatide loaded CD1d-PE tetramers were incubated with either PBS (control), 1D12 or 1D22 (4:1 VHH:C01d ratio) after which tetramers were used to stain Jurkat-Vδ1 cells (final concentration tetramer 2 µg/ml) in combination with CD3-APC. Tetramer binding is prevented by 1D12 and reduced by 1D22 (A). C1R-CD1d cells were incubated with DMSO controls or sulfatide (25 µg/ml) after which medium or anti-CD1d VHH were added at 1000 or 100 nM and incubated for 30 min. Next Jurkat-Vδ1 cells were added and the co-culture was incubated for an additional 24h after which CD69 expression was determined by flow cytometry (B). N=3.

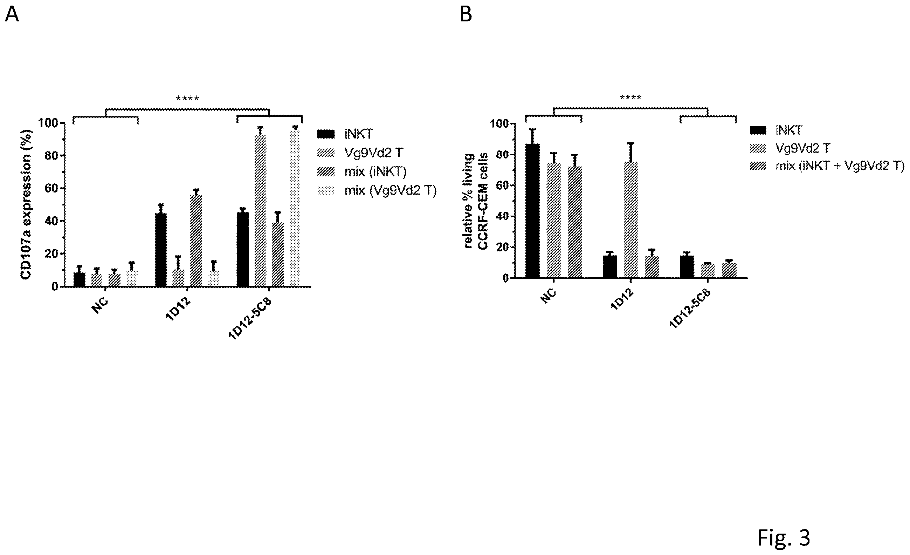

**Fig. 3****.** Dual activation of iNKT cells and Vγ9Vδ2-T cells by a bispecific COld and Vγ9Vδ2-TCR targeting VHH resulting in effector cell degranulation and rapid tumor cell lysis. CD1d-expressing CCRF-CEM cells were incubated with either iNKT cells or Vγ9Vδ2-T cells or both at an effector to target ratio of 1:2 in the presence of medium only, monovalent 1D12 or bispecific 1D12-5C8. Robust degranulation (as depicted by CD107a expression) of iNKT cells in the presence of 1D12 was observed, but only simultaneous degranulation of iNKT cells and Vγ9Vδ2-T cells was seen in the presence of the bispecific VHH (A). In accordance, a striking reduction in living CCRF-CEM cells (B) was observed. Data represent mean+SD of 3 individual experiments with iNKT/ Vγ9Vδ2-T obtained from different donors.

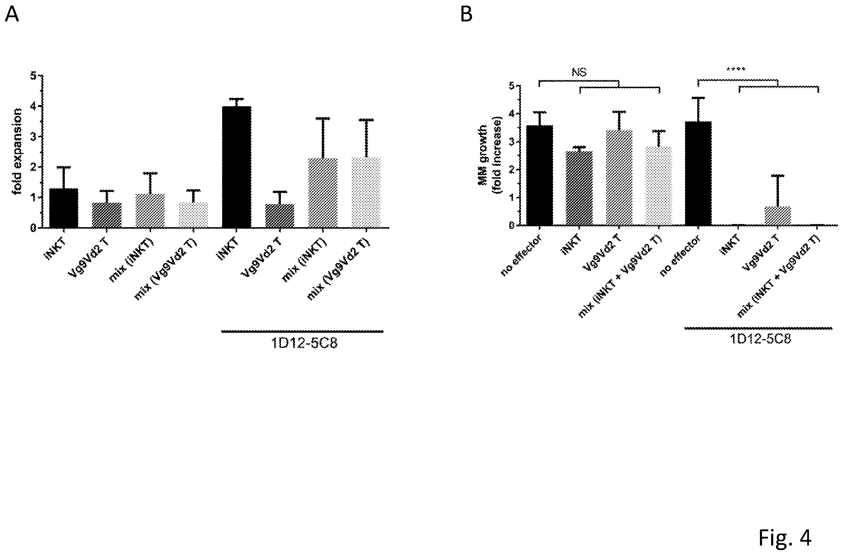

**Fig. 4****.** Bispecific 1D12-5C8 supports iNKT and Vγ9Vδ2-T cell expansion and induces tumor growth control. iNKT, Vγ9Vδ2-T or a mixture (2:3 ratio) were incubated with MM.1s-CD1d cells at an effector to target ratio of 1:10 in the presence of medium only or bispecific 1D12-5C8. Clear induction of iNKT cell expansion was observed, however Vγ9Vδ2-T expansion was only observed in the presence of 1D12-5C8 and iNKT cells (A). Noteworthy, tumor growth was contained in the presence of 1D12-5C8 (B). Data represent mean+SD of 3 individual experiments with paired iNKT/ Vγ9Vδ2-T obtained from different donors.

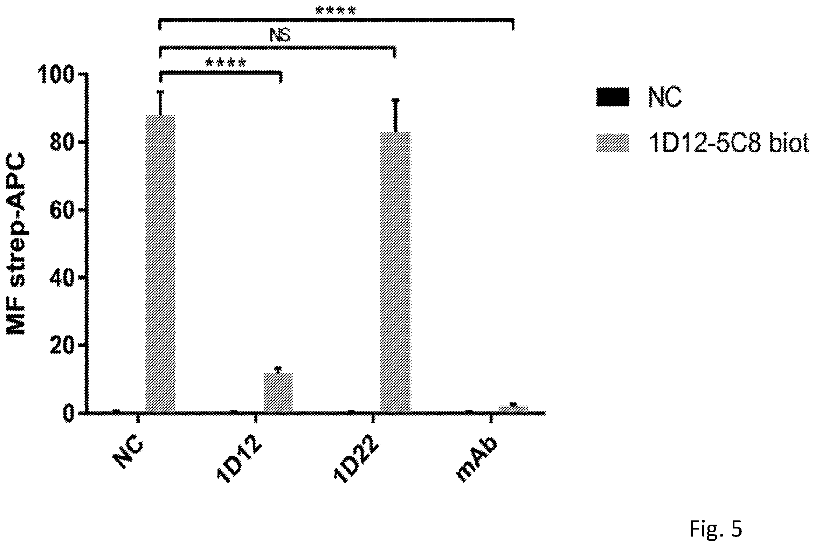

**Fig. 5****.** Anti-CD1d VHH 1D12 and anti-CD1d 51.1 mAb, but not anti-CD1d VHH 1D22, interfere with binding of 1D12-5C8 bispecific VHH. CDId transfected multiple myeloma cells (MM.1s) were incubated with for 45 min with PBS (negative control, NC), anti-CD1d VHH (1000 nM) or anti-CD1d mAb (100 nM) after which PBS (NC) or biotinylated 1D12-5C8 bispecific VHH (100 nM) was added and incubated for an additional 30 min. After extensive washing samples were stained with streptavidin-APC and analyzed by flow cytometry. Data represent mean + SD of 3 individual experiments, ****P < 0.0001, calculated with a two - way analysis of variance with Turkeys's post hoc test.

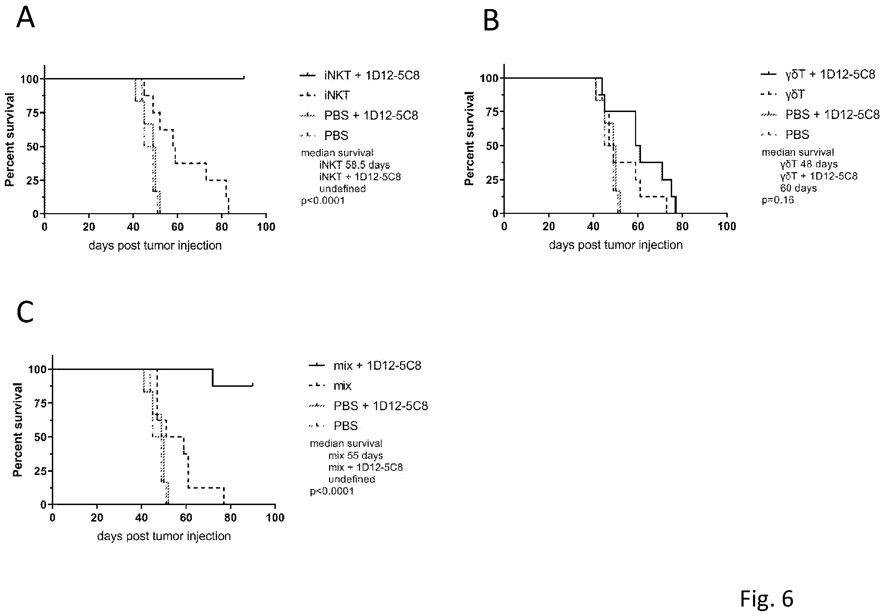

**Fig. 6****.** Bispecific antibody 1D12-5C8 promotes survival in a mouse multiple myeloma model in the presence of iNKT and/or γδ T cells. Panel A shows the effects of administration of antibody 1D12-5C8 and/or iNKT cells on survival. Panel B shows the effects of administration of antibody 1D12-5C8 and/or γδ T cells. Panel C shows the effects of administration of antibody 1D12-5C8 and/or a mixture ("mix") of iNKT and γδ T cells.

### Detailed description of the invention

As described above, in a first main aspect, the invention provides a binding molecule comprising a first binding moiety of an antibody that is able to compete with 1D12 (SEQ ID NO: 4) in binding to a COld molecule and comprising a second binding moiety of an antibody that is able to specifically bind to a Vγ9Vδ2-TCR, wherein the binding molecule is able to activate iNKT cells and wherein the binding molecule is able to activate Vγ9Vδ2 T cells.

Disclosed, but not claimed, is a binding molecule comprising a first binding moiety that is able to compete with single domain antibody 1D12 (SEQ ID NO: 4) in binding to a COld molecule, for use in the treatment of disorders caused, maintained and/or propagated by CD1d-restricted Vδ1+ T-cell activation.

A "disorder caused, maintained and/or propagated by CD1d-restricted Vδ1+ T-cell activation" is a disease or disorder in which CD1d-restricted Vδ1+ T-cell activation initiates, maintains or exacerbates the disease or disorder, or has a negative impact on the prognosis or progression of the disease or disorder. With negative impact is meant that due to the action of the CD1d-restricted Vδ1+ T-cells the disease is sustained, aggravated, or less attenuated compared with the situation where there would be no action of the Vδ1+ T-cells. Preferably, the negative impact is due to (anti-)inflammatory effects of activated Vδ1+ T-cells. Such disorders or diseases in particular include those diseases that require a type 1 helper T (Th1) cell response and are negatively influenced by a T cell response of the type 2 helper T (Th2) cell. The designations "Th1" and "Th2" cells are well known in the art and it is generally believed that Th1 cells are predominantly active towards intracellular microbiota, such as intracellular bacteria and viruses, whereas Th2 cells predominantly activate the humoral immune system (B cells, eosinophils, etc.) in order to combat extracellular microbiota, such as protozoa. Further, Th1 cells promote the killing of tumor cells, whereas Th2 cells counteract such action.

One example of a disorder caused, maintained, and/or propagated by CD1d-restricted Vδ1+ T-cell activation is CD1d-restricted peripheral T cell lymphoma (PTCL (Bachy et al (2016) J Exp Med 213 No. 5: 841)). In a preferred embodiment, a binding molecule is provided for use in the treatment of hematological malignancies, such as CD1d-restricted peripheral T cell lymphoma (PTCL), CD1d+ T-cell acute lymphoblastic leukemia (ALL), CD1d+ lymphoma, CD1d+ chronic lymphocytic leukemia (CLL), CD1d+ acute myelogenous leukemia (AML), and CD1d+ mantle cell lymphoma, preferably for use in the treatment of CD1d-restricted Vδ1+ PTCL, wherein the binding molecule comprises a first binding moiety of an antibody that is able to compete with 1D12 (SEQ ID NO: 4) in binding to a COld molecule and a second binding moiety of an antibody that is able to specifically bind to a Vγ9Vδ2-TCR, wherein the binding molecule is able to activate iNKT cells and wherein the binding molecule is able to activate Vγ9Vδ2 T cells..

The "binding molecule" according to the invention may be any kind of binding molecule, for example a complex, as long as it comprises or consists of a first binding moiety of an antibody and a second binding moiety of an antibody as defined by the invention.

The term "antibody" as used herein is intended to refer to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The binding region (or binding domain) which interacts with an antigen, comprises variable regions of both the heavy and light chains of the immunoglobulin molecule. The constant regions of the antibodies (Abs) may mediate the binding of the immunoglobulin to host tissues or factors, including various cells of the immune system (such as effector cells and T cells) and components of the complement system such as C1q, the first component in the classical pathway of complement activation. In some embodiments, however, the Fc region of the antibody has been modified to become inert, "inert" means an Fc region which is at least not able to bind any Fcy Receptors, induce Fc-mediated cross-linking of FcRs, or induce FcR-mediated cross-linking of target antigens via two Fc regions of individual proteins, such as antibodies. In a further embodiment, the inert Fc region is in addition not able to bind C1q. In one embodiment, the antibody contains mutations at positions 234 and 235 (Canfield and Morrison (1991) J Exp Med 173:1483), e.g. a Leu to Phe mutation at position 234 and a Leu to Glu mutation at position 235. In another embodiment, the antibody contains a Leu to Ala mutation at position 234, a Leu to Ala mutation at position 236 and a Pro to Gly mutation at position 329.

As indicated above, the term antibody as used herein, unless otherwise stated or clearly contradicted by context, includes fragments of an antibody that retain the ability to specifically interact, such as bind, to the antigen. It has been shown that the antigen-binding function of an antibody may be performed by fragments of a full-length antibody. Examples of binding fragments encompassed within the term "antibody" include (i) a Fab' or Fab fragment, a monovalent fragment consisting of the VL, VH, CL and CH1 domains, or a monovalent antibody as described in WO2007059782; (ii) F(ab')2 fragments, bivalent fragments comprising two Fab fragments linked by a disulfide bridge at the hinge region; (iii) a Fd fragment consisting essentially of the VH and CH1 domains; and (iv) a Fv fragment consisting essentially of the VL and VH domains of a single arm of an antibody. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise noted or clearly indicated by context. Although such fragments are generally included within the meaning of antibody, they collectively and each independently are unique features of the present invention, exhibiting different biological properties and utility. These and other useful antibody fragments in the context of the present invention are discussed further herein. It also should be understood that the term antibody, unless specified otherwise, also includes polyclonal antibodies, monoclonal antibodies (mAbs), chimeric antibodies and humanized antibodies, and antibody fragments retaining the ability to specifically bind to the antigen (antigen-binding fragments) provided by any known technique, such as enzymatic cleavage, peptide synthesis, and recombinant techniques. An antibody as generated can possess any isotype.

The term "immunoglobulin heavy chain", "heavy chain of an immunoglobulin" or "heavy chain" as used herein is intended to refer to one of the chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The heavy chain constant region may further comprise a hinge region. The term "immunoglobulin" as used herein is intended to refer to a class of structurally related glycoproteins consisting of two pairs of polypeptide chains, one pair of light (L) chains and one pair of heavy (H) chains, all four potentially inter-connected by disulfide bonds. Within the structure of the immunoglobulin (e.g. IgG), the two heavy chains are inter-connected via disulfide bonds in the so-called "hinge region". Equally to the heavy chains each light chain is typically comprised of several regions; a light chain variable region (abbreviated herein as VL) and a light chain constant region (abbreviated herein as CL). The light chain constant region typically is comprised of one domain, CL. Furthermore, the VH and VL regions may be further subdivided into regions of hypervariability (or hypervariable regions which may be hypervariable in sequence and/or form structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Choitia and Lesk (1987) J. Mol. Biol. 196:901 or Kabat et al. (1991) Sequence of protein of immunological interest, fifth edition. NIH publication. Various methods for CDR determination and amino acid numbering can be compared on www.abysis.org (UCL).

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) [SEQ ID NO:407]) that is encoded by heavy chain constant region genes. Thus, in one embodiment, the antibody comprises a heavy chain of an immunoglobulin of the IgG1 class or any allotype thereof. Further, each heavy chain isotype can be combined with either a kappa (κ) or lambda (λ) light chain.

The term "chimeric antibody" as used herein, refers to an antibody wherein the variable region is derived from a non-human species (e.g. derived from rodents) and the constant region is derived from a different species, such as human. Chimeric antibodies may be generated by antibody engineering. "Antibody engineering" is a generic term used for different kinds of modifications of antibodies, and which is a well-known process for the skilled person. Thus, the chimeric antibody may be a genetically engineered recombinant antibody. Some chimeric antibodies may be both genetically or an enzymatically engineered. It is within the knowledge of the skilled person to generate a chimeric antibody, and thus, generation of the chimeric antibody according to the present invention may be performed by other methods than described herein. Chimeric monoclonal antibodies for therapeutic applications are developed to reduce antibody immunogenicity. They may typically contain non-human (e.g. murine) variable regions, which are specific for the antigen of interest, and human constant antibody heavy and light chain domains. The terms "variable region" or "variable domains" as used in the context of chimeric antibodies, refers to a region which comprises the CDRs and framework regions of both the heavy and light chains of the immunoglobulin.

The term "humanized antibody" as used herein, refers to a genetically engineered non-human antibody, which contains human antibody constant domains and non-human variable domains modified to contain a high level of sequence homology to human variable domains. This can be achieved by grafting of the six non-human antibody complementarity-determining regions (CDRs), which together form the antigen binding site, onto a homologous human acceptor framework region (FR). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and fully human constant regions. Optionally, additional amino acid modifications, which are not necessarily back-mutations, may be applied to obtain a humanized antibody with preferred characteristics, such as affinity and biochemical properties. The amino acid sequence of an antibody of non-human origin is distinct from antibodies of human origin, and therefore a non-human antibody is potentially immunogenic when administered to human patients. However, despite the non-human origin of the antibody, its CDR segments are responsible for the ability of the antibody to bind to its target antigen and humanization aims to maintain the specificity and binding affinity of the antibody. Thus, humanization of non-human therapeutic antibodies is performed to minimize its immunogenicity in man while such humanized antibodies at the same time maintain the specificity and binding affinity of the antibody of non-human origin.

The term "multispecific antibody" refers to an antibody having specificities for at least two different, such as at least three, typically non-overlapping, epitopes. Such epitopes may be on the same or different targets. If the epitopes are on different targets, such targets may be on the same cell or different cells or cell types.

The term "bispecific antibody" refers to an antibody having specificities for at least two different, typically non-overlapping, epitopes. Such epitopes may be on the same or different targets. If the epitopes are on different targets, such targets may be on the same cell or different cells or cell types. In one embodiment, the bispecific antibody comprises a first and a second heavy chain.

Examples of bispecific antibody molecules which may be used in the present invention comprise (i) a single antibody that has two arms comprising different antigen-binding regions, (ii) a single chain antibody that has specificity to two different epitopes, e.g., via two scFvs linked in tandem by an extra peptide linker; (iii) a dual-variable- domain antibody (DVD-IgTM), where each light chain and heavy chain contains two variable domains in tandem through a short peptide linkage; (iv) a chemically-linked bispecific (Fab')2 fragment; (v) a TandAb^{®}, which is a fusion of two single chain diabodies resulting in a tetravalent bispecific antibody that has two binding sites for each of the target antigens; (vi) a flexibody, which is a combination of scFvs with a diabody resulting in a multivalent molecule; (vii) a so called "dock and lock" molecule (Dock-and-Lock^{®}), based on the "dimerization and docking domain" in Protein Kinase A, which, when applied to Fabs, can yield a trivalent bispecific binding protein consisting of two identical Fab fragments linked to a different Fab fragment; (viii) a so-called Scorpion molecule, comprising, e.g., two scFvs fused to both termini of a human Fab-arm; and (ix) a diabody.

Examples of different classes of bispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab- fragments are fused together, fused to heavy-chain constant-domains, Fc-regions or parts thereof; and (vi) ScFv-and diabody-based and heavy chain antibodies (e.g., domain antibodies, Nanobodies^{®}) wherein different single chain Fv molecules or different diabodies or different heavy-chain antibodies (e.g. domain antibodies, Nanobodies^{®}) are fused to each other or to another protein or carrier molecule fused to heavy-chain constant-domains, Fc-regions or parts thereof.

Examples of IgG-like molecules with complementary CH3 domains molecules include but are not limited to the Triomab^{®} (Trion Pharma/Fresenius Biotech), the Knobs-into-Holes (Genentech), CrossMAbs (Roche) and the electrostatically-matched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck,), mAb-Fv (Xencor), bivalent bispecific antibodies (Roche, WO2009080254) and DuoBody^{®} molecules (Genmab).

Examples of recombinant IgG-like dual targeting molecules include but are not limited to Dual Targeting (DT)-Ig (GSK/Domantis, WO2009058383), Two-in-one Antibody (Genentech, Bostrom, et al 2009. Science 323, 1610-1614), Cross-linked Mabs (Karmanos Cancer Center), mAb2 (F-Star), ZybodiesTM (Zyngenia, LaFleur et al. MAbs. 2013 Mar-Apr;5(2):208-18), approaches with common light chain, κλBodies (Novlmmune, WO2012023053) and CovX-body^{®} (CovX/Pfizer, Doppalapudi, V.R., et al 2007. Bioorg. Med. Chem. Lett. 17,501-506).

Examples of IgG fusion molecules include but are not limited to Dual Variable Domain (DVD)-IgTM (Abbott), Dual domain double head antibodies (Unilever; Sanofi Aventis), IgG-like Bispecific (ImClone/Eli Lilly, Lewis et al. Nat Biotechnol. 2014 Feb;32(2):191-8), Ts2Ab (Medlmmune/AZ, Dimasi et al. J Mol Biol. 2009 Oct 30;393(3):672-92) and BsAb (Zymogenetics, WO2010111625), HERCULES (Biogen ldec), scFv fusion (Novartis), scFv fusion (Changzhou Adam Biotech Inc) and TvAb (Roche).

Examples of Fc fusion molecules include but are not limited to ScFv/Fc Fusions (Academic Institution, Pearce et al Biochem Mol Biol Int. 1997 Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock^{®} (DNL) (ImmunoMedics), Bivalent Bispecific (Biotecnol) and Fab-Fv (UCB-Celltech).

Examples of ScFv-, diabody-based and domain antibodies include but are not limited to Bispecific T Cell Engager (BiTE^{®}) (Micromet, Tandem Diabody (Tandab) (Affimed), Dual Affinity Retargeting Technology (DARTTM) (MacroGenics), Single-chain Diabody (Academic, Lawrence FEBS Lett. 1998 Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75), dual targeting nanobodies^{®} (Ablynx, Hmila et al., FASEB J. 2010), dual targeting heavy chain only domain antibodies.

A "binding moiety" in the context of the present invention is a moiety of an antibody that is capable of specifically binding to a target, and, as used herein, is to be understood as referring to a binding moiety of an antibody. A binding moiety may, e.g., comprise: an intact immunoglobulin molecule such as a monoclonal antibody. Alternatively, the binding moiety can comprise an antigen-binding functional fragment, including, but not limited to, Fab, F(ab'), F(ab')2, Fv, dAb, Fd, a complementarity determining region (CDR) fragment, a single chain antibody (scFv), a divalent single chain antibody, a single chain phage antibody, a bispecific double chain antibody, a triabody, a tetrabody, a single domain antibody (nanobody^{®}), a (poly)peptide containing at least an amino acid sequence that is sufficient to specifically bind to its target, and artificial immunoglobulin fragments, such as plastic antibodies (Hoshino et al (2008) J Am Chem Soc 130(46):15242). In a preferred embodiment, a binding moiety is a single domain antibody. Preferably, a binding moiety comprises three heavy chain CDRs.

The term "specifically binds" as used herein, refers to the binding of a binding moiety or binding molecule to a predetermined antigen or target (e.g. human CD1d) to which binding typically is with an affinity corresponding to a K_{D} of about 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less when determined by for instance surface plasmon resonance (SPR) technology in a BIAcore 3000 instrument using the antigen as the ligand and the binding moiety or binding molecule as the analyte, and binds to the predetermined antigen with an affinity corresponding to a K_{D} that is at least ten-fold lower, such as at least 100 fold lower, for instance at least 1,000 fold lower, such as at least 10,000 fold lower, for instance at least 100,000 fold lower than its affinity for binding to a non-specific antigen (e.g., BSA, casein) other than the predetermined antigen or a closely- related antigen. The degree with which the affinity is lower is dependent on the K_{D} of the binding moiety or binding molecule, so that when the K_{D} of the binding moiety or binding molecule is very low (that is, the binding moiety or binding molecule is highly specific), then the degree with which the affinity for the antigen is lower than the affinity for a non-specific antigen may be at least 10,000 fold. The term "K_{D}" (M), as used herein, refers to the dissociation equilibrium constant of a particular interaction between the antigen and the binding moiety or binding molecule.

As mentioned, a binding moiety described above is able to compete for binding to human COld with single domain antibody 1D12. In the context of the present invention, "competition" or "able to compete" or "compete" refers to any detectably significant reduction in the propensity for a particular binding molecule (e.g. a CDId antibody) to bind a particular binding partner (e.g. CD1d) in the presence of another molecule (e.g. a different COld antibody) that binds the binding partner. Typically, competition means an at least about 25 percent reduction, such as an at least about 50 percent, e.g. an at least about 75 percent, such as an at least 90 percent reduction in binding between a CDld binding molecule or moiety, caused by the presence of another CDld binding molecule or moiety as determined by, e.g., ELISA analysis or flow cytometry using sufficient amounts of the two or more competing binding molecules or moieties. Additional methods for determining binding specificity by competitive inhibition may be found in for instance Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc, and Wiley InterScience N. Y, (1992, 1993), and Muller, Meth. Enzymol. 92, 589-601 (1983)). Preferably, the binding molecule of the present invention binds to at least part of or in the vicinity of the same epitope on COld as antibody 1D12, more preferably to the same epitope as 1D12 or to an epitope that overlaps with the epitope of 1D12. Methods for determining the epitope of a binding molecule, such as an antibody are known in the art. With "in the vicinity of" is meant that it binds COld such that the binding molecule at least sterically hinders binding of 1D12 to COld. The binding molecule of the present invention may comprise identical or different CDR sequences as 1D12. In a more preferred embodiment, the binding molecule of the present invention comprises identical CDR1 (SEQ ID NO 1), CDR2 (SEQ ID NO: 2) and CDR3 (SEQ ID NO: 3) sequences as 1D12. In a most preferred embodiment, the binding molecule of the present invention comprises an identical sequence as that of 1D12 (SEQ ID NO: 4) or it comprises the sequence set forth in SEQ ID NO: 85).

As described above, in a main aspect, the invention provides a binding molecule comprising a first binding moiety of an antibody that is able to compete with 1D12 (the antibody set forth in SEQ ID NO: 4) in binding to a COld molecule and comprising a second binding moiety of an antibody that is able to specifically bind to a Vγ9Vδ2-TCR, wherein the binding molecule is able to activate iNKT cells and wherein the binding molecule is able to activate Vγ9Vδ2 T cells. "Specifically binds to a Vγ9Vδ2-TCR" means that the binding molecule binds Vγ9Vδ2-TCR, but does not exclude that the binding molecule binds to one of the separate subunits in the absence of the other subunit, i.e. to the Vy9 chain alone or to the Vδ2 chain alone. For example, as can be seen in Table 2 herein, antibody 5C8 is an antibody that binds the Vγ9Vδ2-TCR, but also binds the Vδ2 chain when the Vδ2 chain is expressed alone.

In a preferred embodiment, the binding molecule binds the Vδ2 chain of Vγ9Vδ2-TCR. Furthermore, preferably, the binding molecule is able to activate Vγ9Vδ2 T cells independently of its natural ligand. The invention provides the insight that such a binding molecule comprising a bispecific immunoglobulin-complex is able to inhibit Vδ1+ T cells and enables activation of (Vγ9)Vδ2+ T cells. Activation of Vγ9Vδ2 T cells is beneficial for the treatment of a plethora of diseases. A Vδ2 TCR chain specific immunoglobulin is preferred over a Vγ9 TCR chain specific immunoglobulin, as Vy9 might pair with, e.g., Vδ1 TCR chain, resulting in attracting and activating a potentially counterproductive γδ-T cell.

The term "first" and "second" binding moiety of an antibody does not refer to their orientation / position in the binding molecule, i.e. it has no meaning with regard to the N- or C-terminus. The term "first" and "second" only serves to correctly and consistently refer to the two different binding moieties in the claims and the description. In one preferred embodiment, the first binding moiety and the second binding moiety are coupled to each other through one or more amide bond(s), preferably through a linker, more preferably comprising the amino acids GGGGS (SEQ ID NO: 83). Two, non-limiting, examples of binding molecules according to the invention comprising a first and a second binding moiety linked through a peptide linker (GGGGS), are depicted in Table 1 (SEQ ID NO: 84 and SEQ ID NO: 87). In another preferred embodiment, the binding molecule is a bispecific antibody, such as a full-length bispecific antibody. The term "full-length antibody" when used herein, refers to an antibody which contains all heavy and light chain constant and variable domains corresponding to those that are normally found in a wild-type antibody of that isotype.

With "able to activate Vγ9Vδ2 T cells" in the context of the present invention is meant that Vγ9Vδ2 T cells are activated in the presence of the binding molecule according to the invention, in particular in the presence of a target cell expressing CD1d. Preferably the activation of the Vγ9Vδ2 T cells is measurable through gene-expression and/or (surface) marker expression (e.g., activation markers, such as CD25, CD69, or CD107a) and/or secretory protein (e.g., cytokines or chemokines) profiles. In a preferred embodiment, the binding molecule is able to induce activation (e.g. upregulation of CD69 and/or CD25 expression) resulting in degranulation (marked by an increase in CD107a expression; Example 3) and cytokine production (e.g. TNFα, IFNy) by Vγ9Vδ2 T cells. Preferably activation of Vγ9Vδ2 T cells takes place in vivo, particularly in a human body that has been administered a binding molecule according to the invention and which human body comprises Vγ9Vδ2 T cells and preferably CD1d+ target cells. Preferably, a binding molecule of the present invention is able to increase CD107a expression on Vγ9Vδ2 T cells to at least 10%, more preferably at least 20%, more preferably at least 40%, most preferably at least 90%, when used in an assay as described in Example 3, wherein e.g. 10% means that 10% of the total number of cells is positive for CD107a. In another embodiment, the number of cells positive for CD107a is increased 1.5-fold, such as 2-fold, e.g. 5-fold, in the presence of a binding molecule of the invention.

Similarly, for iNKT cells, "able to activate" in the context of the present invention means that iNKT cells behave differently in the presence of a binding molecule or a binding molecule for use according to the invention, in particular in the presence of a CDld molecule, preferably in the presence of a COld molecule on a cell surface. Markers, such as CD25 (Example 1), CD69, CD107a (Example 3), or cytokines / chemokines, such as IFNy (Example 1), TNFα, IL-2, are used to determine whether iNKT cells are activated. Preferably the activation of iNKT cells takes place in vivo, particularly in a human body that has been administered a binding molecule according to the invention and which human body comprises iNKT cells and preferably CD1d+ target cells. With CD1d+ target cells are meant CD 1d+ cells that contribute to disease pathogenicity and not normal CD1d-expressing cells. Preferably a binding molecule of the present invention is able to increase CD107a expression on iNKT cells to at least 20%, more preferably to least 30%, most preferably to least 40%, when used in an assay as described in Example 3, wherein e.g. 10% means that 10% of the total number of cells is positive for CD107a. In another embodiment, the number of cells positive for CD107a is increased 1.5-fold, such as 2-fold, e.g. 5-fold, in the presence of a binding molecule of the invention. Furthermore, preferably a binding molecule of the present invention is able to increase CD25 expression on iNKT cells to at least 10 fold, more preferably to at least 20 fold, most preferably to at least 30 fold compared to a "vehicle" control, as measured mean fluorescence intensity by flow cytometry, using allophycocyanin (APC) -conjugated CD25 in a FACS, when used in an assay as described in Example 1. Preferably, a binding molecule of the present invention is able to increase IFNy expression by iNKT cells at least 1.5-fold, such as at least 2-fold or at least 3-fold, when used in an assay as described in Example 1.

The bispecific (or multispecific) binding molecule preferably induces a Th1 type response. In a preferred embodiment, a binding molecule comprising a first and a second binding moiety according to the invention is provided for use as a medicament, preferably for use in the treatment of a tumor. With tumor within the context of the present invention is meant: An abnormal mass of tissue, either in one location (e.g. solid tumors) or distributed over the human body (e.g. metastases). In addition, abnormal mass of tissue is also meant to refer to disseminated tumors (e.g. liquid hematological tumors). Tumors are also a classical sign of inflammation. However, such inflammation (non-cancerous) tumors and other non-malignant tumors are not included within the definition here. In a preferred embodiment, the tumor is a cancer, especially one with the potential to cause death. Treatment of tumors is specific to the location and type of the tumor. Benign tumors can sometimes simply be ignored, or they may be reduced in size (debulked) or removed entirely via surgery. For malignant or some benign tumors, options include chemotherapy, radiation, and surgery. Tumors of the hematopoietic and lymphoid tissues (so called blood tumors) are also included and comprise, *inter alia,* leukemias, such as ALL, AML, CLL, small lymphocytic lymphoma (SLL), chronic myelogenous leukemia (CML), and acute monocytic leukemia (AMoL), lymphomas, such as Hodgkin's lymphomas and Non-Hodgkin's lymphomas, and myelomas.

In a preferred embodiment, a binding molecule comprising a first and a second binding moiety according to the invention is provided, wherein the binding molecule is able to reduce Vδ1 T cell activation. Reducing Vδ1 T cell activation in this context means that a Vδ1 T cell is no longer able to recognize its ligand on a COld molecule that is bound to a binding molecule as defined in the invention. Such reduced Vδ1 T cell activation can, e.g., be determined by measuring the expression of activation marker CD69 on Vδ1+ T cells. Lower CD69 expression levels are observed in less activated Vδ1+ T cells, e.g., Jurkat cells as used in Example 2. In particular when used in the context of Vδ1+ tumor cells, blocking means that the presence of the binding molecule according to the invention has a negative impact on tumor cell growth and/or viability. Preferably, CD69 expression on Jurkat cells is increased less than 5-fold, such as less than 2-fold by a binding molecule according to the invention, as compared to "vehicle" control when tested in an assay as described in Example 2.

The binding molecule according to the invention is able to activate iNKT cells. As said before, the inventors have shown that 1D12 is able to activate iNKT cells, irrespective of the presence of an exogenous ligand for iNKT cells, such as α-galactosylceramide. The present disclosure further provides the insight that COld recognition by Vδ1+ T cells can be blocked by such binding molecules and that the presence of a second binding moiety of an antibody in a binding molecule according to the invention enables the activation of Vδ2+ T cells. Such triple acting binding molecule, i.e. capable of reducing activation of CD1d-restricted Vδ1+ T cells and activating iNKT cells, and at the same time allowing activation of Vδ2+ T cells, create a micro-environment that is skewed towards a Th1-type anti-tumor response. The reduced activation of Vδ1+ T cells, the activation of iNKT cells, and the activation of Vδ2+ T cells synergize towards a tumor-aggressive micro-environment promoting effective tumor-cell killing.

A binding molecule according to the invention is thus modified such that it not only reduces activation of Vδ1+ T cells and activates iNKT cells, but also binds and activates Vδ2+ T cells through clustering of Vγ9Vδ2-TCR on γδ-T cells. For this, a second binding moiety of an antibody is encompassed within the binding molecule according to the invention. This second binding moiety is able to bind to a Vγ9Vδ2-TCR, preferably to the Vδ2 chain. Several such antibodies, which bind to the Vδ2 chain or the Vy9 chain of the TCR have been described in WO2015156673 and are depicted in Table 1 and Table 2. In a further aspect, the invention provides a first binding molecule according to the invention comprising a first binding moiety of an antibody and a second binding moiety of an antibody, wherein the first moiety binds to CDId and the second binding moiety binds to a Vγ9Vδ2-TCR, wherein the binding molecule is able to activate iNKT cells and wherein the binding molecule is able to activate Vγ9Vδ2 T cells, for use in combination with a second binding molecule, comprising a binding moiety that binds to CD1d, but not comprising a binding moiety that binds to Vγ9Vδ2-TCR. Such a combination treatment might in particular be useful if the first and second binding molecules have their effects at very different concentrations. E.g. an excess of a CDld antibody which blocks at high concentration could be combined with an CD1d/ Vγ9Vδ2 antibody which activates γδ-T cells at low concentration.

**Table 1 Designation of VHH (CDR), TCR chains, and sequences of the various VHHs comprised within a binding molecule according to the invention.**

| SEQ ID. | code | Description | Sequence |
|---|---|---|---|
| 1 | 1D12 | CDR1 | DNVMG |
| 2 | 1D12 | CDR2 | TIRTGGSTNYADSVKG |
| 3 | 1D12 | CDR3 | TIPVPSTPYDY |
| 4 | 1D12 | | QVQLVESGGGLVQAGGSLRLSCAASGSMFSDNVM GWYRQAPGKQRELVATIRTGGSTNYADSVKGRFT ISRDNAKNTVYLQMNSLKPEDTAVYYCRHTIPVP STPYDYWGQGTQVTVSS |
| 5 | 5E3 | CDR1 | SYAMG |
| 6 | 5E3 | CDR2 | AISWSGGTTYYADSVKG |
| 7 | 5E3 | CDR3 | SLDCSGPGCHTAEYDY |
| 8 | 6H1 | CDR1 | EYAMG |
| 9 | 6H1 | CDR2 | AISWTGSKTYYADSVKG |
| 10 | 6H1 | CDR3 | SSDCSGPGCHTEEYDY |
| 11 | 5G3 | CDR1 | SYAMG |
| 12 | 5G3 | CDR2 | AVSWSGGSTYYADSVKG |
| 13 | 5G3 | CDR3 | SQDCSGPGCYTNEYDS |
| 14 | 5C1 | CDR1 | NYAMA |
| 15 | 5C1 | CDR2 | AVSWSGGRTYYADSVKG |
| 16 | 5C1 | CDR3 | SLSCSGPGCSLEEYDY |
| 17 | 5D3 | CDR1 | NYAMG |
| 18 | 5D3 | CDR2 | VISWSGGSTYYADSVKG |
| 19 | 5D3 | CDR3 | QFSGASTVVAGTALDYDY |
| 20 | 6E3 | CDR1 | NYGMG |
| 21 | 6E3 | CDR2 | GISWSGGSTDYADSVKG |
| 22 | 6E3 | CDR3 | VFSGAETAYYPSDDYDY |
| 23 | 6H4 | CDR1 | NYGMG |
| 24 | 6H4 | CDR2 | GISWSGGSTDYADSVKG |
| 25 | 6H4 | CDR3 | VFSGAETAYYPSDDYDY |
| 26 | 6C1 | CDR1 | NYGMG |
| 27 | 6C1 | CDR2 | GISWSGGSTDYADSVKG |
| 28 | 6C1 | CDR3 | VFSGAETAYYPSDDYDY |
| 29 | 6H3 | CDR1 | NYGMG |
| 30 | 6H3 | CDR2 | GITWSGGSTHYADLVKG |
| 31 | 6H3 | CDR3 | VFSGAETAYYPSTEYDY |
| 32 | 6G3 | CDR1 | NYGMG |
| 33 | 6G3 | CDR2 | GISWSGGSTYYADSVKG |
| 34 | 6G3 | CDR3 | VFSGAETAQYPSYDYDY |
| 35 | 5C8 | CDR1 | NYAMG |
| 36 | 5C8 | CDR2 | AISWSGGSTSYADSVKG |
| 37 | 5C8 | CDR3 | QFSGADYGFGRLGIRGYEYDY |
| 38 | 5F5 | CDR1 | NYAMG |
| 39 | 5F5 | CDR2 | AISWSGGSTYYADSVKG |
| 40 | 5F5 | CDR3 | MFSGSESQLVVVITNLYEYDY |
| 41 | 6A1 | CDR1 | NYAMG |
| 42 | 6A1 | CDR2 | TISWSGGSTYYADSVKG |
| 43 | 6A1 | CDR3 | AFSGSDYANTKKEVEYDY |
| 44 | 6E4 | CDR1 | DYCIA |
| 45 | 6E4 | CDR2 | CITTSDGSTYYADSVKG |
| 46 | 6E4 | CDR3 | YFGYGCYGGAQDYRAMDY |
| 47 | 5C7 | CDR1 | RYTMG |
| 48 | 5C7 | CDR2 | AISWSGGRTNFAGSVKG |
| 49 | 5C7 | CDR3 | DWLPVPGRESYDY |
| 50 | 5D7 | CDR1 | NYAMG |
| 51 | 5D7 | CDR2 | AISWSGGMTDHADSVKG |
| 52 | 5D7 | CDR3 | AFAGDIPYGSSWYGDPTTYDY |
| 53 | 5B11 | CDR1 | TFSMA |
| 54 | 5B11 | CDR2 | AINWSGGSTRYADSVSD |
| 55 | 5B11 | CDR3 | RRGGIYYSTQNDYDY |
| 56 | 6C4 | CDR1 | DYRMG |
| 57 | 6C4 | CDR2 | TISWSGGLTYYADSVKG |
| 58 | 6C4 | CDR3 | GGGYAGGTYYHPEE |
| 59 | 5E3 | VHH | EVQLVESGGGLVQAGGSLRLSCTASGRTFSSYAM GWFRQAPGKEREFVAAISWSGGTTYYADSVKGRF |
| | | | TISRDNAKNTVSLQMNSLKPEDTAVYFCAASLDC SGPGCHTAEYDYWGQGTQVTVSS |
| 60 | 6H1 | VHH | EVQLVESGGGLVQAGGSLRLSCAATGRTFSEYAM GWFRQAPGKEREFAAAISWTGSKTYYADSVKGRF TISRDNAKNTVYLQMNSLKPEDTAVYYCAASSDC SGPGCHTEEYDYWGQGTQVTVSS |
| 61 | 5G3 | VHH | EVQLVESGGGLVQAGGSLRLSCAASGRTFSSYAM GWFRQAPGKEREFVAAVSWSGGSTYYADSVKGRF TISRDNARNTVYLQMNSLNPEDTAVYYCAASQDC SGPGCYTNEYDSWGQGTQVTVSS |
| 62 | 5C1 | VHH | EVQLVESGGGLVQPGGSLRLSCAASGSIFSNYAM AWFRQAPEKERDFLAAVSWSGGRTYYADSVKGRF TISRDNAKNTVNLQMNSLKPEDTAVYYCAASLSC SGPGCSLEEYDYWGQGTQVTVSS |
| 63 | 5D3 | VHH | EVQLVESGGGLVQAGGSLRLSCAASGRPFSNYAM GWFRQAPGKEREFVTVISWSGGSTYYADSVKGRF TISRDNAKNTVYLQMNSLKPEDTAVYYCAAQFSG ASTVVAGTALDYDYWGQGTRVTVSS |
| 64 | 6E3 | VHH | EVQLVESGGGLVQAGGSLRLSCAASGRPFSNYGM GWFRQAPGKKREFVAGISWSGGSTDYADSVKGRL TISRDNAKNTVYLQMNSLKPEDTAVYYCAAVFSG AETAYYPSDDYDYWGQGTQVTVSS |
| 65 | 6H4 | VHH | EVQLVESGGGLVQAGGSLRLSCAASGRPFSNYGM GWFRQAPGKKREFVAGISWSGGSTDYADSVKGRF TISRDNAKNTVYLQMNSLKPEDTAVYYCAAVFSG AETAYYPSDDYDYWGQGTQVTVSS |
| 66 | 6C1 | VHH | EVQLVESGGGLVQAGGSLRLSCAASGRPFSNYGM GWFRQAPGKKRESVAGISWSGGSTDYADSVKGRF TISRDNAKNTVYLQMNSLKPEDTAVYYCAAVFSG |
| | | | AETAYYPSDDYDYWGQGTQVTVSS |
| 67 | 6H3 | VHH | EVQLVESGGGLVQAGGSLRLSCAVSGRPFSNYGM GWFRQAPGKEREFVAGITWSGGSTHYADLVKGRF TISRDNAKNTVHLQMNSLKPEDTAVYYCAAVFSG AETAYYPSTEYDYWGQGTQVTVSS |
| 68 | 6G3 | VHH | EVQLVESGGGLVQAGGSLRLSCAASGRPFNNYGM GWFRQAPGKEREFVAGISWSGGSTYYADSVKGRF TISRDNAKNTVYLQMNSLKPEDTAVYYCAAVFSG AETAQYPSYDYDYWGQGTQVTVSS |
| 69 | 5C8 | VHH | EVQLVESGGGLVQAGGSLRLSCAASGRPFSNYAM GWFRQAPGKEREFVAAISWSGGSTSYADSVKGRF TISRDNAKNTVYLQMNSPKPEDTAIYYCAAQFSG ADYGFGRLGIRGYEYDYWGQGTQVTVSS |
| 70 | 5F5 | VHH | EVQLVESGGGLVQAGGSLRLSCAASGRTFSNYAM GWFRQAPGKEREFVAAISWSGGSTYYADSVKGRF TISRDNAKNTVYLQMNSLKPEDTAVYYCAAMFSG SESQLVVVITNLYEYDYWGQGTQVTVSS |
| 71 | 6A1 | VHH | EVQLVESGGGLVQAGGSLRLSCAASGRTFSNYAM GWFRQAPGKEREFVATISWSGGSTYYADSVKGRF TISRDNAKNTVYLQMNSLKPEDTAVYYCAAAFSG SDYANTKKEVEYDYWGQGTQVTVSS |
| 72 | 6E4 | VHH | EVQLVESGGGLVQAGGSLRLSCAASGFTFDDYCI AWFRQAPGKEREPVSCITTSDGSTYYADSVKGRF TISSDNAKNTVYLQMNRLKPEDTAVYYCAAYFGY GCYGGAQDYRAMDYWGKGTLVTVSS |
| 73 | 5C7 | VHH | EVQLVESGGGLVQAGDSLRLSCAASGRTFSRYTM GWFRQAPGKEREFVAAISWSGGRTNFAGSVKGRF TISRDNAKNTVYLQMNSLKPEDTAVYYCAADWLP VPGRESYDYWGQGTQVTVSS |
| 74 | 5D7 | VHH | EVQLVESGGGLVQAGGSLRLSCIASGRTFSNYAM GWFRQAPGKEREFVAAISWSGGMTDHADSVKGRF TISRDNAKNTVYLQMNSLKPEDTAVYYCAAAFAG DIPYGSSWYGDPTTYDYWGQGTQVTVSS |
| 75 | 5B11 | VHH | EVQLVESGGGLVQAGGSLRLSCAASGRTSSTFSM AWFRQAPRKEREFVAAINWSGGSTRYADSVSDRF AISRDNAKNTVYLQMNNLKPEDTAVYYCAARRGG IYYSTQNDYDYWGQGTQVTVSS |
| 76 | 6C4 | VHH | EVQLVESGGGLVQAGGSLRLSCAVSVRTFSDYRM GWFRQAPGKEREFVSTISWSGGLTYYADSVKGRF TISRDNSKNTLYLQMNSLKPEDTAVYYCAAGGGY AGGTYYHPEEWGQGTQVTVSS |
| 77 | Human Vγ9 chain | TCR | MLSLLHASTLAVLGALCVYGAGHLEQPQISSTKT LSKTARLECVVSGITISATSVYWYRERPGEVIQF LVSISYDGTVRKESGIPSGKFEVDRIPETSTSTL TIHNVEKQDIATYYCALWEAQQELGKKIKVFGPG TKLIITDKQLDADVSPKPTIFLPSIAETKLQKAG TYLCLLEKFFPDVIKIHWEEKKSNTILGSQEGNT MKTNDTYMKFSWLTVPEKSLDKEHRCIVRHENNK NGVDQEIIFPPIKTDVITMDPKDNCSKDANDTLL LQLTNTSAYYMYLLLLLKSVVYFAIITCCLLRRT AFCCNGEKS |
| 78 | Human Vδ2 chain | TCR | |
| | | | |
| 79 | 1D22 | CDR1 | NAMG |
| 80 | 1D22 | CDR2 | VISSSGSTNYADSVKG |
| 81 | 1D22 | CDR3 | HVAGFDEYNY |
| 82 | 1D22 | VHH | QVQLVESGGGLVQAGGSLRLSCAASGSIFSINAM GWYRQAPGKQRDFLAVISSSGSTNYADSVKGRFT ISRDNAKNTAYLQMNSLKVEDTAVYYCAAHVAGF DEYNYWGQGTQVTVSS |
| 83 | GS-linker | Linker | GGGGS |
| 84 | 1D12-5C8 | Bispecific binding molecule | QVQLVESGGGLVQAGGSLRLSCAASGSMFSDNVM GWYRQAPGKQRELVATIRTGGSTNYADSVKGRFT ISRDNAKNTVYLQMNSLKPEDTAVYYCRHTIPVP STPYDYWGQGTQVTVSSGGGGSEVQLVESGGGLV QAGGSLRLSCAASGRPFSNYAMGWFRQAPGKERE FVAAISWSGGSTSYADSVKGRFTISRDNAKNTVY LQMNSPKPEDTAIYYCAAQFSGADYGFGRLGIRG YEYDYWGQGTQVTVSS |
| 85 | 1D12 var | VHH | EVQLVESGGGLVQAGGSLRLSCAASGSMFSDNVM GWYRQAPGKQRELVATIRTGGSTNYADSVKGRFT ISRDNAKNTVYLQMNSLKPEDTAVYYCRHTIPVP STPYDYWGQGTQVTVSS |
| 86 | 5C8 var 1 | VHH | EVQLLESGGGSVQPGGSLRLSCAASGRPFSNYAM SWFRQAPGKEREFVSAISWSGGSTSYADSVKGRF TISRDNSKNTLYLQMNSLRAEDTAVYYCAAQFSG ADYGFGRLGIRGYEYDYWGQGTQVTVSS |
| 87 | 1D12-5C8 var | Bispecific binding molecule | EVQLVESGGGLVQAGGSLRLSCAASGSMFSDNVM |
| | | | GWYRQAPGKQRELVATIRTGGSTNYADSVKGRFT ISRDNAKNTVYLQMNSLKPEDTAVYYCRHTIPVP STPYDYWGQGTQVTVSSGGGGSEVQLLESGGGSV QPGGSLRLSCAASGRPFSNYAMSWFRQAPGKERE FVSAISWSGGSTSYADSVKGRFTISRDNSKNTLY LQMNSLRAEDTAVYYCAAQFSGADYGFGRLGIRG YEYDYWGQGTQVTVSS |
| 88 | 5C8 var 2 | VHH | EVQLLESGGGLVQPGGSLRLSCAASGRPFSNYAM SWFRQAPGKEREFVSAISWSGGSTSYADSVKGRF TISRDNSKNTLYLQMNSLRAEDTAVYYCAAQFSG ADYGFGRLGIRGYEYDYWGQGTLVTVSS |

**Table 2: From WO2015156673: Binding of VHHs to γδ T- cells expressing a Vy9 or Vδ2 chain (paired with a non Vδ2 or Vy9 chain respectively), expressing the complete Vγ9Vδ2 TCR, or not expressing any of the Vγ9Vδ2 TCR chains. "-" indicates a Mean Fluorescence Index (MFI) below 1.5, "+/-" indicates an MFI of between 1.5 and 4.5, "+" indicates an MFI of between 4.6 and 20 and "++" indicates an MFI above 20.**

| SEQ ID | Ref | Vδ2 + | Vγ9 + | Vγ9Vδ2 + | Vγ9Vδ2 - |
|---|---|---|---|---|---|
| 59 | 5E3 | - | ++ | ++ | - |
| 60 | 6H1 | - | ++ | ++ | - |
| 61 | 5G3 | - | ++ | ++ | - |
| 62 | 5C1 | +/- | ++ | ++ | - |
| 63 | 5D3 | ++ | - | ++ | - |
| 64 | 6E3 | ++ | - | ++ | - |
| 65 | 6H4 | ++ | - | ++ | - |
| 66 | 6C1 | ++ | - | ++ | - |
| 67 | 6H3 | ++ | +/- | ++ | - |
| 68 | 6G3 | ++ | - | ++ | - |
| 69 | 5C8 | ++ | - | ++ | - |
| 70 | 5F5 | ++ | - | ++ | - |
| 71 | 6A1 | ++ | - | ++ | - |
| 72 | 6E4 | ++ | - | ++ | - |
| 73 | 5C7 | +/- | - | +/- | - |
| 74 | 5D7 | ++ | - | ++ | - |
| 75 | 5B11 | - | - | + | - |
| 76 | 6C4 | +/- | ++ | ++ | - |

Thus, further provided is a binding molecule according to the invention, or a binding molecule for use according to the invention, wherein the second binding moiety is able to compete with binding to a Vγ9Vδ2-TCR with single domain antibody 5E3 (SEQ ID NO: 59), 6H1 (SEQ ID NO: 60), 5G3 (SEQ ID NO: 61), 5C1 (SEQ ID NO: 62), 5D3 (SEQ ID NO: 63), 6E3 (SEQ ID NO: 64), 6H4 (SEQ ID NO: 65), 6C1 (SEQ ID NO: 66), 6H3 (SEQ ID NO: 67), 6G3 (SEQ ID NO: 68), 5C8 (SEQ ID NO: 69), 5F5 (SEQ ID NO: 70), 6A1 (SEQ ID NO: 71), 6E4 (SEQ ID NO: 72), 5C7 (SEQ ID NO: 73), 5D7 (SEQ ID NO: 74), 5B11 (SEQ ID NO: 75), or 6C4 (SEQ ID NO: 76), preferably with single domain antibody 5E3 (SEQ ID NO: 59), 6H1 (SEQ ID NO: 60), 5G3 (SEQ ID NO: 61), 5C1 (SEQ ID NO: 62), 5D3 (SEQ ID NO: 63), 6E3 (SEQ ID NO: 64), 6H4 (SEQ ID NO: 65), 6C1 (SEQ ID NO: 66), 6H3 (SEQ ID NO: 67), 6G3 (SEQ ID NO: 68), 5C8 (SEQ ID NO: 69), 5F5 (SEQ ID NO: 70), 6A1 (SEQ ID NO: 71), or 6E4 (SEQ ID NO: 72). Preferably, the second binding moiety binds to the same or partly overlapping, more preferably the same epitope sequence as recognized by binding moiety 5E3, 6H1, 5G3, 5C1, 5D3, 6E3, 6H4, 6C1, 6H3, 6G3, 5C8, 5F5, 6A1, 6E4, 5C7, 5D7, 5B11 or 6C4, preferably as recognized by binding moiety 5E3, 6H1, 5G3, 5C1, 5D3, 6E3, 6H4, 6C1, 6H3, 6G3, 5C8, 5F5, 6A1, or 6E4.

In some embodiments of the binding molecule according to the invention or the binding molecule for use according to the invention, the first binding moiety or the second binding moiety, or both, is a single domain antibody. Single domain antibodies (sdAb, also called Nanobody^{®}, or VHH) are well known to the skilled person. Single domain antibodies comprise a single CDR1, a single CDR2 and a single CDR3. Examples of single domain antibodies are variable fragments of heavy chain only antibodies, antibodies that naturally do not comprise light chains, single domain antibodies derived from conventional antibodies, and engineered antibodies. Single domain antibodies may be derived from any species including mouse, human, camel, llama, shark, goat, rabbit, and cow. For example, naturally occurring VHH molecules can be derived from antibodies raised in Camelidae species, for example in camel, dromedary, alpaca and guanaco.

Like a whole antibody, a single domain antibody is able to bind selectively to a specific antigen. Single domain antibodies may contain only the variable domain of an immunoglobulin chain, i.e. CDR1, CDR2 and CDR3 and framework regions. With a molecular weight of only about 12-15 kDa, single domain antibodies are much smaller than common antibodies (150-160 kDa) which are composed of two heavy chains and two light chains or even Fab fragments (53 kDa), composed of one light chain and part of a heavy chain. The format of a single domain antibody has the advantage of less steric hindering when bound to its target.

In a preferred embodiment, a binding molecule according to the invention or a binding molecule for use according to the invention is provided, wherein the first binding moiety comprises a CDR1 sequence GSMFSDNVMG (SEQ ID NO: 1), a CDR2 sequence TIRTGGSTNYADSVKG (SEQ ID NO: 2), and/or a CDR3 sequence TIPVPSTPYDY (SEQ ID NO: 3), or any of those sequences wherein, independently, any of the amino acids, preferably at most 4 amino acids, more preferably at most 3, more preferably at most 2, most preferably at most 1, have been substituted, preferably conservatively substituted according to table 3.

In a further preferred embodiment, a binding molecule according to the invention or a binding molecule for use according to the invention is provided, wherein the first binding moiety comprises the sequence set forth in SEQ ID NO: 4. or the sequence set forth in SEQ ID NO: 85

**Table 3 conservative amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gin |
| Asn | Gln; His | Met | Leu; Ile |
| Asp | Glu | Phe | Met; Leu; Tyr |
| Gln | Asn | Ser | Thr, Gly |
| Cys | Ser | Thr | Ser; Val |
| Glu | Asp | Trp | Tyr |
| Gly | Pro | Tyr | Trp; Phe |
| His | Asn; Gin | Val | Ile; Leu |
| Ile | Leu, Val | | |

In a preferred embodiment, a binding molecule according to the invention or a binding molecule for use according to the invention is provided, wherein the second binding moiety comprises a CDR 1, CDR2 and CDR3 sequence in the combinations shown in table 1 for each VHH. More specifically, the second binding moiety comprises a CDR 1 sequence according SEQ ID NO: 5, a CDR 2 sequence according to SEQ ID NO: 6 and/or a CDR 3 sequence according to SEQ ID NO: 7; or a CDR 1 sequence according SEQ ID NO: 8, a CDR 2 sequence according to SEQ ID NO: 9 and/or a CDR 3 sequence according to SEQ ID NO: 10; or a CDR 1 sequence according SEQ ID NO: 11, a CDR 2 sequence according to SEQ ID NO: 12 and/or a CDR 3 sequence according to SEQ ID NO: 13; or a CDR 1 sequence according SEQ ID NO: 14, a CDR 2 sequence according to SEQ ID NO: 15 and/or a CDR 3 sequence according to SEQ ID NO: 16; or a CDR 1 sequence according SEQ ID NO: 17, a CDR 2 sequence according to SEQ ID NO: 18 and/or a CDR 3 sequence according to SEQ ID NO: 19; or a CDR 1 sequence according SEQ ID NO: 20, a CDR 2 sequence according to SEQ ID NO: 21 and/or a CDR 3 sequence according to SEQ ID NO: 22; or a CDR 1 sequence according SEQ ID NO: 23, a CDR 2 sequence according to SEQ ID NO: 24 and/or a CDR 3 sequence according to SEQ ID NO: 25; or a CDR 1 sequence according SEQ ID NO: 26, a CDR 2 sequence according to SEQ ID NO: 27 and/or a CDR 3 sequence according to SEQ ID NO: 28; or a CDR 1 sequence according SEQ ID NO: 29, a CDR 2 sequence according to SEQ ID NO: 30 and/or a CDR 3 sequence according to SEQ ID NO: 31; or a CDR 1 sequence according SEQ ID NO: 32, a CDR 2 sequence according to SEQ ID NO: 33 and/or a CDR 3 sequence according to SEQ ID NO: 34; or a CDR 1 sequence according SEQ ID NO: 35, a CDR 2 sequence according to SEQ ID NO: 36 and/or a CDR 3 sequence according to SEQ ID NO: 37; or a CDR 1 sequence according SEQ ID NO: 38, a CDR 2 sequence according to SEQ ID NO: 39 and/or a CDR 3 sequence according to SEQ ID NO: 40; or a CDR 1 sequence according SEQ ID NO: 41, a CDR 2 sequence according to SEQ ID NO: 42 and/or a CDR 3 sequence according to SEQ ID NO: 43; or a CDR 1 sequence according SEQ ID NO: 44, a CDR 2 sequence according to SEQ ID NO: 45 and/or a CDR 3 sequence according to SEQ ID NO: 46; or a CDR 1 sequence according SEQ ID NO: 47, a CDR 2 sequence according to SEQ ID NO: 48 and/or a CDR 3 sequence according to SEQ ID NO: 49; or a CDR 1 sequence according SEQ ID NO: 50, a CDR 2 sequence according to SEQ ID NO: 51 and/or a CDR 3 sequence according to SEQ ID NO: 52; or a CDR 1 sequence according SEQ ID NO: 53, a CDR 2 sequence according to SEQ ID NO: 54 and/or a CDR 3 sequence according to SEQ ID NO: 55; or a CDR 1 sequence according SEQ ID NO: 56, a CDR 2 sequence according to SEQ ID NO: 57 and/or a CDR 3 sequence according to SEQ ID NO: 58, or any of those sequences wherein, independently, any of the amino acids, preferably at most 4 amino acids, more preferably at most 3, more preferably at most 2, most preferably at most 1, has been conservatively substituted, according to table 3.

More preferred, the second binding moiety comprises a CDR 1 sequence according SEQ ID NO: 5, a CDR 2 sequence according to SEQ ID NO: 6 and/or a CDR 3 sequence according to SEQ ID NO: 7; or a CDR 1 sequence according SEQ ID NO: 8, a CDR 2 sequence according to SEQ ID NO: 9 and/or a CDR 3 sequence according to SEQ ID NO: 10; or a CDR 1 sequence according SEQ ID NO: 11, a CDR 2 sequence according to SEQ ID NO: 12 and/or a CDR 3 sequence according to SEQ ID NO: 13; or a CDR 1 sequence according SEQ ID NO: 14, a CDR 2 sequence according to SEQ ID NO: 15 and/or a CDR 3 sequence according to SEQ ID NO: 16; or a CDR 1 sequence according SEQ ID NO: 17, a CDR 2 sequence according to SEQ ID NO: 18 and/or a CDR 3 sequence according to SEQ ID NO: 19; or a CDR 1 sequence according SEQ ID NO: 20, a CDR 2 sequence according to SEQ ID NO: 21 and/or a CDR 3 sequence according to SEQ ID NO: 22; or a CDR 1 sequence according SEQ ID NO: 23, a CDR 2 sequence according to SEQ ID NO: 24 and/or a CDR 3 sequence according to SEQ ID NO: 25; or a CDR 1 sequence according SEQ ID NO: 26, a CDR 2 sequence according to SEQ ID NO: 27 and/or a CDR 3 sequence according to SEQ ID NO: 28; or a CDR 1 sequence according SEQ ID NO: 29, a CDR 2 sequence according to SEQ ID NO: 30 and/or a CDR 3 sequence according to SEQ ID NO: 31; or a CDR 1 sequence according SEQ ID NO: 32, a CDR 2 sequence according to SEQ ID NO: 33 and/or a CDR 3 sequence according to SEQ ID NO: 34; or a CDR 1 sequence according SEQ ID NO: 35, a CDR 2 sequence according to SEQ ID NO: 36 and/or a CDR 3 sequence according to SEQ ID NO: 37; or a CDR 1 sequence according SEQ ID NO: 38, a CDR 2 sequence according to SEQ ID NO: 39 and/or a CDR 3 sequence according to SEQ ID NO: 40; or a CDR 1 sequence according SEQ ID NO: 41, a CDR 2 sequence according to SEQ ID NO: 42 and/or a CDR 3 sequence according to SEQ ID NO: 43; or a CDR 1 sequence according SEQ ID NO: 44, a CDR 2 sequence according to SEQ ID NO: 45 and/or a CDR 3 sequence according to SEQ ID NO: 46, or any of those sequences wherein, independently, any of the amino acids, preferably at most 4 amino acids, more preferably at most 3, more preferably at most 2, most preferably at most 1, has been conservatively substituted, according to table 3.

In a preferred embodiment, a binding molecule according to the invention is provided, comprising a first binding moiety of an antibody capable of specially binding to CD1d, wherein the first binding moiety comprises a CDR1 sequence according to SEQ ID NO: 1, a CDR2 sequence according to SEQ ID NO: 2 and a CDR3 sequence according to SEQ ID NO: 3.

In a preferred embodiment, a binding molecule comprising a first binding moiety of an antibody capable of specifically binding to CDId and comprising a second binding moiety of an antibody that is able to specifically bind to a Vγ9Vδ2-TCR according to the invention is provided, wherein
- the first binding moiety comprises a CDR1 sequence according to SEQ ID NO: 1, a CDR2 sequence according to SEQ ID NO: 2 and a CDR3 sequence according to SEQ ID NO: 3, and
- the second binding moiety comprises a CDR 1 sequence according SEQ ID NO: 5, a CDR 2 sequence according to SEQ ID NO: 6 and a CDR 3 sequence according to SEQ ID NO: 7; or a CDR 1 sequence according SEQ ID NO: 8, a CDR 2 sequence according to SEQ ID NO: 9 and a CDR 3 sequence according to SEQ ID NO: 10; or a CDR 1 sequence according SEQ ID NO: 11, a CDR 2 sequence according to SEQ ID NO: 12 and a CDR 3 sequence according to SEQ ID NO: 13; or a CDR 1 sequence according SEQ ID NO: 14, a CDR 2 sequence according to SEQ ID NO: 15 and a CDR 3 sequence according to SEQ ID NO: 16; or a CDR 1 sequence according SEQ ID NO: 17, a CDR 2 sequence according to SEQ ID NO: 18 and a CDR 3 sequence according to SEQ ID NO: 19; or a CDR 1 sequence according SEQ ID NO: 20, a CDR 2 sequence according to SEQ ID NO: 21 and a CDR 3 sequence according to SEQ ID NO: 22; or a CDR 1 sequence according SEQ ID NO: 23, a CDR 2 sequence according to SEQ ID NO: 24 and a CDR 3 sequence according to SEQ ID NO: 25; or a CDR 1 sequence according SEQ ID NO: 26, a CDR 2 sequence according to SEQ ID NO: 27 and a CDR 3 sequence according to SEQ ID NO: 28; or a CDR 1 sequence according SEQ ID NO: 29, a CDR 2 sequence according to SEQ ID NO: 30 and a CDR 3 sequence according to SEQ ID NO: 31; or a CDR 1 sequence according SEQ ID NO: 32, a CDR 2 sequence according to SEQ ID NO: 33 and a CDR 3 sequence according to SEQ ID NO: 34; or a CDR 1 sequence according SEQ ID NO: 35, a CDR 2 sequence according to SEQ ID NO: 36 and a CDR 3 sequence according to SEQ ID NO: 37; or a CDR 1 sequence according SEQ ID NO: 38, a CDR 2 sequence according to SEQ ID NO: 39 and a CDR 3 sequence according to SEQ ID NO: 40; or a CDR 1 sequence according SEQ ID NO: 41, a CDR 2 sequence according to SEQ ID NO: 42 and a CDR 3 sequence according to SEQ ID NO: 43; or a CDR 1 sequence according SEQ ID NO: 44, a CDR 2 sequence according to SEQ ID NO: 45 and a CDR 3 sequence according to SEQ ID NO: 46; or a CDR 1 sequence according SEQ ID NO: 47, a CDR 2 sequence according to SEQ ID NO: 48 and a CDR 3 sequence according to SEQ ID NO: 49; or a CDR 1 sequence according SEQ ID NO: 50, a CDR 2 sequence according to SEQ ID NO: 51 and a CDR 3 sequence according to SEQ ID NO: 52; or a CDR 1 sequence according SEQ ID NO: 53, a CDR 2 sequence according to SEQ ID NO: 54 and a CDR 3 sequence according to SEQ ID NO: 55; or a CDR 1 sequence according SEQ ID NO: 56, a CDR 2 sequence according to SEQ ID NO: 57 and a CDR 3 sequence according to SEQ ID NO: 58.

In one embodiment, the invention relates to a binding molecule comprising a first binding moiety of an antibody capable of specifically binding to CDId and comprising a second binding moiety of an antibody that is able to specifically bind to a Vγ9Vδ2-TCR wherein the binding molecule is able to activate iNKT cells and wherein the binding molecule is able to activate Vγ9Vδ2 T cells, for use in the treatment of disorders caused, maintained and/or propagated by CD1d-restricted Vδ1+T-cell activation, preferably for use in the treatment of CD1d-restricted Vδ1+ peripheral T cell lymphoma, wherein
- the first binding moiety comprises a CDR1 sequence according to SEQ ID NO: 1, a CDR2 sequence according to SEQ ID NO: 2 and a CDR3 sequence according to SEQ ID NO: 3, and
- the second binding moiety comprises a CDR 1 sequence according SEQ ID NO: 5, a CDR 2 sequence according to SEQ ID NO: 6 and a CDR 3 sequence according to SEQ ID NO: 7; or a CDR 1 sequence according SEQ ID NO: 8, a CDR 2 sequence according to SEQ ID NO: 9 and a CDR 3 sequence according to SEQ ID NO: 10; or a CDR 1 sequence according SEQ ID NO: 11, a CDR 2 sequence according to SEQ ID NO: 12 and a CDR 3 sequence according to SEQ ID NO: 13; or a CDR 1 sequence according SEQ ID NO: 14, a CDR 2 sequence according to SEQ ID NO: 15 and a CDR 3 sequence according to SEQ ID NO: 16; or a CDR 1 sequence according SEQ ID NO: 17, a CDR 2 sequence according to SEQ ID NO: 18 and a CDR 3 sequence according to SEQ ID NO: 19; or a CDR 1 sequence according SEQ ID NO: 20, a CDR 2 sequence according to SEQ ID NO: 21 and a CDR 3 sequence according to SEQ ID NO: 22; or a CDR 1 sequence according SEQ ID NO: 23, a CDR 2 sequence according to SEQ ID NO: 24 and a CDR 3 sequence according to SEQ ID NO: 25; or a CDR 1 sequence according SEQ ID NO: 26, a CDR 2 sequence according to SEQ ID NO: 27 and a CDR 3 sequence according to SEQ ID NO: 28; or a CDR 1 sequence according SEQ ID NO: 29, a CDR 2 sequence according to SEQ ID NO: 30 and a CDR 3 sequence according to SEQ ID NO: 31; or a CDR 1 sequence according SEQ ID NO: 32, a CDR 2 sequence according to SEQ ID NO: 33 and a CDR 3 sequence according to SEQ ID NO: 34; or a CDR 1 sequence according SEQ ID NO: 35, a CDR 2 sequence according to SEQ ID NO: 36 and a CDR 3 sequence according to SEQ ID NO: 37; or a CDR 1 sequence according SEQ ID NO: 38, a CDR 2 sequence according to SEQ ID NO: 39 and a CDR 3 sequence according to SEQ ID NO: 40; or a CDR 1 sequence according SEQ ID NO: 41, a CDR 2 sequence according to SEQ ID NO: 42 and a CDR 3 sequence according to SEQ ID NO: 43; or a CDR 1 sequence according SEQ ID NO: 44, a CDR 2 sequence according to SEQ ID NO: 45 and a CDR 3 sequence according to SEQ ID NO: 46; or a CDR 1 sequence according SEQ ID NO: 47, a CDR 2 sequence according to SEQ ID NO: 48 and a CDR 3 sequence according to SEQ ID NO: 49; or a CDR 1 sequence according SEQ ID NO: 50, a CDR 2 sequence according to SEQ ID NO: 51 and a CDR 3 sequence according to SEQ ID NO: 52; or a CDR 1 sequence according SEQ ID NO: 53, a CDR 2 sequence according to SEQ ID NO: 54 and a CDR 3 sequence according to SEQ ID NO: 55; or a CDR 1 sequence according SEQ ID NO: 56, a CDR 2 sequence according to SEQ ID NO: 57 and a CDR 3 sequence according to SEQ ID NO: 58.

In a more preferred embodiment the second binding moiety comprised by the binding molecule of the invention, comprises a sequence selected from any of the SEQ ID NOs: 59 - 76, 86 or 88, or any of those sequences wherein, independently, any of the amino acids, preferably at most 20 amino acids, more preferably at most 15, more preferably at most 10, more preferably at most 5, more preferably at most 4, more preferably at most 3, more preferably at most 2, most preferably at most 1, has been substituted, preferably conservatively substituted, according to table 3.

In a most preferred embodiment the second binding moiety comprised by the binding molecule of the invention, comprises a sequence selected from any of the SEQ ID NOs: 59 - 72, 86 or 88, or any of those sequences wherein, independently, any of the amino acids, preferably at most 20 amino acids, more preferably at most 15, more preferably at most 10, more preferably at most 5, more preferably at most 4, more preferably at most 3, more preferably at most 2, most preferably at most 1, has been substituted, preferably conservatively substituted, according to table 3.

In a further preferred embodiment, the first binding moiety comprised by the binding molecule of the invention, comprises the sequence set forth in SEQ ID NO: 85 and the second binding moiety comprises the sequence set forth in SEQ ID NO: 86.

In a further preferred embodiment, the first binding moiety comprised by the binding molecule of the invention, comprises the sequence set forth in SEQ ID NO: 85 and the second binding moiety comprises the sequence set forth in SEQ ID NO: 88.

In a further preferred embodiment, the binding molecule comprises or consists of the sequence set forth in SEQ ID NO: 87.

CDR1, CDR2 and CDR3 sequences or framework regions may be exchanged between species. For example, from a llama immunoglobulin molecule, CDR sequences may be selected and exchanged with CDR sequences in a human immunoglobulin molecule, to obtain a human immunoglobulin molecule having the specificity that is derived from the llama CDR sequences. This may be advantageous as a human sequence may be less immunogenic to humans as compared an antibody containing the original llama framework sequence. Such an exchange of sequences is known as humanization. Hence, the immunoglobulin molecules as provided by the invention may have human derived immunoglobulin sequences or immunoglobulin sequences derived from other animals, such as but not limited to: camelid, llama, shark, and have the CDR1, CDR2 and CDR3 sequences replaced with the CDR sequences according to the invention in order to provide for human CDld binding. In other words, the binding molecule according to the invention may comprise a humanized single-domain antibody with CDRs as disclosed herein. For example, a single domain antibody may have human framework sequences and CDR regions as disclosed herein.

The invention thus provides a binding molecule that enables activation of iNKT cells and at the same time reduces activation of Vδ1+ T cells. In a preferred embodiment, a binding molecule according to the invention or a binding molecule for use according to the invention is provided, wherein the binding molecule further comprises a tumor-targeting moiety. The tumor-targeting moiety comprises a binding moiety, able to specifically bind to a tumor antigen. The binding molecule also comprises a binding moiety that is capable of binding Vγ9Vδ2-TCR and is preferably able to compete with binding to a Vγ9Vδ2-TCR with any of the VHHs depicted in Table 1.

Tumor antigens are proteins that are produced by tumor cells that elicit an immune response, particularly T-cell mediated immune responses. The selection of the antigen binding moiety of the invention will depend on the particular type of cancer to be treated. Tumor antigens are well known in the art and include, for example a glioma-associated antigen, carcinoembryonic antigen (CEA), EGFRvIII, Interleukin- 11 receptor alpha (IL-I IRα), Interleukin- 13 receptor subunit alpha-2 (IL-13Ra or CD213A2), epidermal growth factor receptor (EGFR), B7H3 (CD276), Kit (CD117), carbonic anhydrase (CA-IX), CS-1 (also referred to as CD2 subset 1), Mucin 1, cell surface associated (MUC1), BCMA, oncogene fusion protein consisting of breakpoint cluster region (BCR) and Abelson murine leukemia viral oncogene homolog 1 (Abl) bcr-abl, Receptor tyro sine-protein kinase ERBB2 (HER2/neu), β-human chorionic gonadotropin, alphafetoprotein (AFP), anaplastic lymphoma kinase (ALK), CD19, CD123, cyclin Bl, lectin-reactive AFP, Fos-related antigen 1, adrenoceptor beta 3 (ADRB3), thyroglobulin, tyrosinase; ephrin type-A receptor 2 (EphA2), Receptor for Advanced Glycation Endproducts (RAGE-1), renal ubiquitous 1 (RU1), renal ubiquitous 2 (RU2), synovial sarcoma, X breakpoint 2 (SSX2), A kinase anchor protein 4 (AKAP-4), lymphocyte-specific protein tyrosine kinase (LCK), proacrosin binding protein sp32 (OY-TES1), Paired box protein Pax-5 (PAX5), Squamous Cell Carcinoma Antigen Recognized By T Cells 3 (SART3), C-type lectin-like molecule- 1 (CLL-1 or CLECL1), fucosyl GM1, hexasaccharide portion of globoH glycoceramide (GloboH), MN-CA IX, Epithelial cell adhesion molecule (EPCAM), EVT6-AML, transglutaminase 5 (TGSS), human telomerase reverse transcriptase (hTERT), polysialic acid, placenta-specific 1 (PLAC1), intestinal carboxyl esterase, LewisY antigen, sialyl Lewis adhesion molecule (sLe), lymphocyte antigen 6 complex, locus K 9 (LY6K), heat shock protein 70-2 mutated (mut hsp70-2), M-CSF, v-myc avian, myelocytomatosis viral oncogene neuroblastoma derived homolog (MYCN), Ras Homolog Family Member C (RhoC), Tyrosinase-related protein 2 (TRP-2), Cytochrome P450 1B1 (CYP1B1), CCCTC-Binding Factor (Zinc Finger Protein)-Like (BORIS or Brother of the Regulator of Imprinted Sites), prostase, prostate- specific antigen (PSA), paired box protein Pax-3 (PAX3), prostatic acid phosphatase (PAP), Cancer/testis antigen 1 (NY-ESO-1), Cancer/testis antigen 2 (LAGE-Ia), LMP2, neural cell adhesion molecule (NCAM), tumor protein p53 (p53), p53 mutant, Rat sarcoma (Ras) mutant, glycoprotein 100 ( gplOO), prostein, OR51E2, pannexin 3 (PANX3), pro state- specific membrane antigen (PSMA), prostate stem cell antigen (PSCA),high molecular weight-melanoma-associated antigen (HMWMAA), Hepatitis A virus cellular receptor 1 (HAVCRI), vascular endothelial growth factor receptor 2 (VEGFR2), Platelet-derived growth factor receptor beta (PDGFR-beta), legumain, human papilloma virus E6 (HPV E6), human papilloma virus E7 (HPV E7), survivin, telomerase, sperm protein 17 (SPA17), Stage- specific embryonic antigen-4 (SSEA-4), tyrosinase, TCR Gamma Alternate Reading Frame Protein (TARP), Wilms tumor protein (WT1), prostate-carcinoma tumor antigen- 1 (PCTA-1), melanoma inhibitor of apoptosis (ML-IAP), MAGE, Melanoma-associated antigen 1 (MAGE- Al), melanoma cancer testis antigen-1 (MAD-CT-1), melanoma cancer testis antigen-2 (MAD- CT-2), melanoma antigen recognized by T cells 1 (MelanA/MARTI), X Antigen Family, Member 1A (XAGE1), elongation factor 2 mutated (ELF2M), ERG (TMPRSS2 ETS fusion gene), N-Acetyl glucosaminyl-transferase V (NA17), neutrophil elastase, sarcoma translocation breakpoints, mammary gland differentiation antigen (NY-BR-1), ephrinB2, CD20, CD22, CD24, CD30, CD33, CD38, CD44v6, CD97, CD171, CD179a, androgen receptor, insulin growth factor (IGF)-I, IGF-II, IGF-I receptor, ganglioside GD2 (GD2), o-acetyl-GD2 ganglioside (OAcGD2), ganglioside GD3 (aNeu5Ac(2-8)aNeu5Ac(2-3)bDGalp(I -4)bDGlcp(I-I)Cer), ganglioside GM3 (aNeuSAc(2-3)bDGa3p(I-4)bDGlcp(I-I)Cer), G protein-coupled receptor class C group 5, member D (GPRCSD), G protein-coupled receptor 20 (GPR20), chromosome X open reading frame 61 (CXORF61), folate receptor (FRα), folate receptor beta, Receptor tyrosine kinase-like orphan receptor 1 (ROR1), Fms-Like Tyrosine Kinase 3 (Flt3), Tumor-associated glycoprotein 72 (TAG72), Tn antigen (TN Ag or (GaINAca-Ser/Thr)), angiopoietin-binding cell surface receptor 2 (Tie 2), tumor endothelial marker 1 (TEM1 or CD248), tumor endothelial marker 7- related (TEM7R), claudin 6 (CLDN6), thyroid stimulating hormone receptor (TSHR), uroplakin 2 (UPK2), mesothelin, Protease Serine 21 (Testisin or PRSS21), epidermal growth factor receptor (EGFR), fibroblast activation protein alpha (FAP), Olfactory receptor 51E2 (OR51E2), ETS translocation-variant gene 6, located on chromosome 12p (ETV6-AML), CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module- containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); folate receptor (FRα); mesothelin; EGFR variant III (EGFRvIII); B-cell maturation antigen (BCMA); GD2; CLL-1; CA-IX; MUC1; HER2; and any combination thereof. In one preferred embodiment, the tumor antigen is selected from the group consisting of folate receptor (FRα), mesothelin, EGFRvIII, IL-13Ra, CD123, CD19, CD33, BCMA, GD2, CLL-1, CA-IX, MUC1, HER2, and any combination thereof. In one embodiment, the tumor targeting moiety is an immunoglobulin that specifically binds to PD-L1, EGFR, CD40, Her2, PSMA, MUC-1, CEA, c-met, CD19, CD20, BCMA, Her3, AFP, CAIX, or CD38.

As already mentioned, a binding molecule according to the invention enables creating a microenvironment that is beneficial for tumor cell killing by, e.g., iNKT cells and Vγ9Vδ2 T cells. A binding molecule comprising a first binding moiety of an antibody and a second binding moiety of an antibody according to the invention for use in the treatment of a tumor is, therefore provided. Such binding molecule is not only effective against CD1d+ tumors, but also against tumors that themselves do not express COld but (in part) rely on CD1d+ suppressive cells (e.g. MDSCs or tumour-associated macrophages (TAMs)) in the tumor environment. Preferably the tumor is selected from hematological malignancies, such as T cell lymphoma, multiple myeloma, acute myeloid leukemia, acute lymphoblastic leukemia, chronic lymphocytic leukemia, chronic myeloid leukemia, mantle cell lymphoma, B cell lymphoma, smoldering myeloma, non-Hodgkin lymphoma, Hodgkin lymphoma, myelomonocytic leukemias, lymphoplasmacytic lymphoma, hairy cell leukemia, and splenic marginal zone lymphoma, or solid tumors, such as renal cell carcinoma, melanoma, colorectal carcinoma, head and neck cancer, breast cancer, prostate cancer, lung cancer, pancreatic cancer, gastro-esophageal cancer, small bowel carcinoma, central nervous system tumors, medulloblastomas, hepatocellular carcinoma, ovarian cancer, glioma, neuroblastoma, Urothelial carcinomas, bladder cancer, sarcoma, penile cancer, basal cell carcinoma, merkel cell carcinoma, neuroendocrine carcinoma, neuroendocrine tumors, carcinoma of unknown primary (CUP), thymoma, vulvar cancer, cervical carcinoma, testicular cancer, cholangiocarcinoma, appendicular carcinoma, mesothelioma, ampullary carcinoma, anal cancer, and choriocarcinoma.

### Pharmaceutical compositions, dosages, modes of administration and methods of treatment

In a further main aspect, the invention relates to a pharmaceutical composition comprising
- a binding molecule, such as an antibody, comprising a first binding moiety of an antibody that is able to compete with antibody 1D12 in binding to a COld molecule and comprising a second binding moiety of an antibody that is able to specifically bind to a Vγ9Vδ2-TCR, wherein the binding molecule is able to activate iNKT cells and wherein the binding molecule is able to activate Vγ9Vδ2 T cells, and
- a pharmaceutically acceptable excipient.

Polypeptides, such as antibodies may be formulated with pharmaceutically acceptable carriers or diluents as well as any other known adjuvants and excipients in accordance with conventional techniques such as those disclosed in (Rowe et al., Handbook of Pharmaceutical Excipients, 2012 June, ISBN 9780857110275). The pharmaceutically acceptable carriers or diluents as well as any other known adjuvants and excipients should be suitable for the polypeptides or antibodies and the chosen mode of administration. Suitability for carriers and other components of pharmaceutical compositions is determined based on the lack of significant negative impact on the desired biological properties of the chosen compound or pharmaceutical composition of the present invention (e.g., less than a substantial impact (10% or less relative inhibition, 5% or less relative inhibition, etc.) upon antigen binding). A pharmaceutical composition may also include diluents, fillers, salts, buffers, detergents (e.g., a nonionic detergent, such as Tween-20 or Tween-80), stabilizers (e.g., sugars or protein-free amino acids), preservatives, tissue fixatives, solubilizers, and/or other materials suitable for inclusion in a pharmaceutical composition. Further pharmaceutically acceptable excipients and carriers include any and all suitable solvents, dispersion media, coatings, antibacterial and antifungal agents, isotonicity agents, antioxidants and absorption-delaying agents, and the like that are physiologically compatible with a binding molecule of the present invention.

The disclosure describes, but does not claim, methods of treating a disease or disorder comprising administering binding molecules as defined herein to a subject in need thereof. The subject may be human. The described, but not claimed, method involves administering an effective amount of the binding molecules. "Treatment" or "treating" refers to the administration of an effective amount of a therapeutically active polypeptide according to the present invention with the purpose of easing, ameliorating, arresting or eradicating (curing) symptoms or disease states. An "effective amount" or "therapeutically effective amount" refers to an amount effective, at dosages and for periods of time necessary, to achieve a desired therapeutic result. A therapeutically effective amount of a polypeptide, such as an antibody, may vary according to factors such as the disease stage, age, sex, and weight of the individual, and the ability of the antibody to elicit a desired response in the individual. A therapeutically effective amount is also one in which any toxic or detrimental effects of the antibody or antibody portion are outweighed by the therapeutically beneficial effects.

Administration may be carried out by any suitable route, but will typically be parenteral, such as intravenous, intramuscular or subcutaneous. Effective dosages and the dosage regimens for the binding molecule, e.g. an antibody, depend on the disease or condition to be treated and may be determined by the persons skilled in the art.

Binding molecules, such as antibodies of the present invention may also be administered in combination therapy, i.e., combined with other therapeutic agents relevant for the disease or condition to be treated. Accordingly, in one embodiment, the antibody-containing medicament is for combination with one or more further therapeutic agents, such as cytotoxic, chemotherapeutic or anti-angiogenic agents. Such combined administration may be simultaneous, separate or sequential. The disclosure describes, but does not claim, a method for treating or preventing disease, such as cancer, which method comprises administration to a subject in need thereof of a therapeutically effective amount of a binding molecule or a pharmaceutical composition of the present invention, in combination with radiotherapy and/or surgery.

### Further aspects and embodiments of the invention

In one aspect, the invention provides a binding molecule comprising a first binding moiety of an antibody that is able to specifically bind to a COld molecule and comprising a second binding moiety of an antibody that is able to specifically bind to a Vγ9Vδ2-TCR, wherein the binding molecule is able to reduce Vδ1 T cell activation, is able to activate iNKT cells, and is able to activate Vγ9Vδ2 T cells.

In a further aspect, the invention relates to an antibody, such as a bispecific antibody, comprising a first binding moiety of an antibody that is able to compete with 1D12 in binding to a COld molecule and comprising a second binding moiety of an antibody that is able to specifically bind to a Vγ9Vδ2-TCR, wherein the antibody is able to activate iNKT cells and wherein the antibody is able to activate Vγ9Vδ2 T cells. Preferably, the antibody is able to reduce Vδ1 T cell activation. Preferably, the first and/or second binding moiety is a single domain antibody.

### Methods of preparing binding molecules of the invention

Binding molecules of the invention, such as polypeptides, in particular antibodies, comprising a first binding moiety of an antibody and a second binding moiety of an antibody, are typically produced recombinantly, i.e. by expression of nucleic acid constructs encoding the polypeptides in suitable host cells, followed by purification of the produced recombinant polypeptide from the cell culture. Nucleic acid constructs can be produced by standard molecular biological techniques well-known in the art. The constructs are typically introduced into the host cell using a vector. Suitable nucleic acid constructs, vectors are known in the art. Host cells suitable for the recombinant expression of polypeptides, such as antibodies are well-known in the art, and include CHO, HEK-293, Expi293F, PER-C6, NS/0 and Sp2/0 cells.

### Examples

### Materials

### Cell lines

The human Epstein-Barr virus-transformed B-lymphoblast cell line C1R, stably transduced with CD1d, and the human cell line JY were grown in Iscove's modified Dulbecco's medium (catalogue no. 12-722F; Lonza, Basel, Switzerland) supplemented with 10% (v/v) fetal calf serum (catalogue no. SV30160.03; HyClone GE Healthcare, Chalfont, St Giles, UK), 0.05 mm β-mercaptoethanol, 100 IU/ml sodium penicillin, 100 µg/ml streptomycin sulphate and 2.0 mm I-glutamine (catalogue no. 10378-016; Life Technologies, Carlsbad, CA). The human cervical adenocarcinoma cell line HeLa, stably transduced with CD1d, was cultured in Dulbecco's modified Eagle's medium (catalogue no. BE12-709F; Lonza) supplemented with 10% (v/v) fetal calf serum, 0.05 mm β-mercaptoethanol, 100 IU/ml sodium penicillin, 100 µg/ml streptomycin sulphate and 2.0 mm I-glutamine. The human myeloma cell line MM.1s with or without mcherry/luc and, stably transduced with CD1d, the human acute T-lymphoblastic leukemia cell line CCRF-CEM, the human acute T cell leukemia cell line Jurkat transduced with a Vδ1 sulfatide-CD1d restricted TCR, and the human acute myeloid leukemia cell lines MOLM-13 and NOMO-1 were cultured in RPMI-1640 (catalogue no. BE12-115F; Lonza) medium supplemented with 10% (v/v) fetal calf serum, 0.05 mm β-mercaptoethanol, 100 IU/ml sodium penicillin, 100 µg/ml streptomycin sulphate and 2.0 mm I-glutamine. CCRF-CEM and MM1.s genetic characteristics were determined by PCR-single-locus-technology and found identical to the published DNA-profiles. Cells were tested mycoplasma-negative and frequently tested for purity (transfectants) by flow cytometry.

### Flow cytometry and monoclonal antibodies

The following antibodies were used in this study: fluorescein isothiocyanate (FITC) conjugated Vδ2, FITC CD69, phycoerythrin (PE) and allophycocyanin (APC) -conjugated CD25 (catalogue nos 555432 and #340907), and APC CD3 were purchased from BD Biosciences (Franklin Lakes, NJ). Phycoerythrin-Cyanine 7-conjugated Vα24 (catalogue no. PN A66907) and Vβ11 PE (catalogue no. IM2290) were purchased from Beckman Coulter (Brea, CA). 7-aminoactinomycin D (7-AAD) was purchased from Sigma (St Louis, MO), PE Vy9 from Biolegend (San Diego, USA), PE CD107a from Miltenyi (Miltenyi Biotec, Bergisch Gladbach, Germany) and FITC annexin V from VPS Diagnostics (Hoever, the Netherlands) (catalogue no. A700). Tetramers were made in house. Flow cytometry staining was performed in FACS buffer (PBS supplemented with 0.1% BSA and 0.02% sodium azide) for 30 min at 4°, unless otherwise specified. Samples were analyzed on FACS Fortessa (BD Biosciences).

### Generation of DC, iNKT and Vγ9Vδ2-T cell lines

moDC and primary human iNKT and γδ T cells were generated as described previously (De Bruin et al (2016) Clin Immunol 169:128). Briefly, monocytes were isolated from peripheral blood mononuclear cells with the use of CD14 MicroBeads (Miltenyi Biotec, Bergisch Gladbach, Germany) and cultured in complete RPMI-1640 medium in the presence of 1000 U/ml granulocyte-macrophage colony-stimulating factor (Sanofi Leukine, Bridgewater, NJ) and 20 ng/ml IL-4 (catalogue no. 204-IL/CF; R&D Systems, Minneapolis, MN) for 5-7 days and subsequently matured with 100 ng/ml lipopolysaccharide (LPS) (catalogue no. L6529; Sigma) in the presence or absence of 100 ng/ml α-GalCer (catalogue no. KRN7000; Funakoshi, Tokyo, Japan) for 48-72 hr. iNKT cells were purified from peripheral blood mononuclear cells of healthy volunteers using magnetic bead sorting, and stimulated weekly with mature α-GalCer-loaded moDC in Yssel's medium supplemented with 1% human AB serum, 10 U/ml IL-7 (catalogue no. 207-IL/CF; R&D Systems) and 10 ng/ml IL-15 (catalogue no. 34-8159; eBioscience). γδT cells were purified from peripheral blood mononuclear cells of healthy volunteers using magnetic bead sorting, and stimulated weekly with pamidronate (10 µM)

(PCH, Pharmachemie BV, Haarlem, The Netherlands) loaded moDC in Yssel's medium supplemented with 1% human AB serum, 100 U/ml IL-2 (BioVision, Mountain View, California, USA) 10 U/ml IL-7 and 10 ng/ml IL-15. Alternatively, γδ T cells were weekly stimulated with irradiated feeders cells (1×10⁶ mixed PBMCs of two donors and 0.1×10⁶ JY cells), 10 IU/mL rhIL-7, 10 µg/mL rhlL-15 and 50 ng/mL PHA in in RPMI-1640 medium supplemented as described above. Depending on culture density, cultured cells were split and fresh culture medium was added. Pure (> 95% Vα24+ Vβ11+ or Vy9+ Vδ2+) iNKT and γδT cells were used for experiments.

### Generation of anti-CD1d and anti-γδTCR specific VHH

The anti-CD1d and anti-γδTCR specific VHH were identified and generated as described previously (Lameris R et al (2016) Immunology 149(1):111; De Bruin et al (2016) Clin Immunol 169:128). Tag-less 1D12, 1D22 and 1D12-5C8 was produced by UPE (Utrecht, the Netherlands).

### In vivo xenograft mouse multiple myeloma (MM) model

A disseminated MM model was established by intravenous transfer of CD1d⁺ MM cells into NOD *scid* gamma (NSG) mice. Female 18-26-week-old NSG mice (Charles River) were irradiated with 2 Gy 24hr prior to intravenous (i.v.) injection of 2.5×10⁶ MM.1s.mcherry/luc.CD1d cells via the tail vain (day 0). On day 7, 14 and 21, 1×10⁷ human iNKT cells, human γδ T cells or a mixture thereof (1:1 ratio) were i.v. injected. Mice were biweekly intraperitoneally (i.p.) injected with PBS or bispecific antibody 1D12-5C8 (100 µg/mouse). Mice were euthanized when pre-set human end-points were reached. Animal experiments were approved by the Dutch Central Authority for Scientific Procedures on Animals (CCD).

### Example 1

### Modulation of iNKT cell activation

To evaluate the capacity of 1D12 and 1D22 to stimulate or inhibit iNKT cell activation, 5×10⁴ Hela-CD1d cells were seeded per well in a 96-well tissue culture plate and pulsed overnight with vehicle control (DMSO 0.01%) or 100 ng/ml α-GalCer. Cells were then washed with PBS and incubated with medium, or the anti-CD1d specific VHH for 1 hr at the indicated concentrations. Subsequently, 5×10⁴ pure and resting iNKT were added to each well. After 24 hr, culture supernatants were analyzed for (induction or inhibition of) cytokine production by CBA (BD Biosciences) whereas iNKT cells were harvested and analyzed for CD25 expression by flow cytometry. As can be seen in figure 1, we identified an anti-CD1d VHH (clone 1D22) that completely blocked iNKT cell activation and cytokine production (P < 0.0001) and thus recognition of the CD1d-α-GalCer complex. In sharp contrast anti-CD1d VHH clone 1D12 was found to potentiate CD1d-restricted iNKT cell activation even in the absence of exogenously added glycolipid Ag (fig. 1 a and c) (P < 0.0001).

### Example 2

### Modulation of Jurkat-Vδ1 cell activation

iNKT cells are known to dock over the extreme F' pocket of CDld which contrasts with sulfatide-CD1d restricted Vδ1-T cells that dock more towards the A' pocket. We therefore evaluated the effect of 1D12 and 1D22 on sulfatide-CD1d restricted Vδ1-T cells. To evaluate the effect of 1D12 and 1D22 on Jurkat-Vδ1 cell activation, 1×10⁵ C1R-CD1d cells were seeded per well in a 96-well tissue culture plate and pulsed for 2 hr with vehicle control (DMSO 0.05%) or 25 µg/ml sulfatide. Cells were then incubated with medium, or the anti-CD1d specific VHH for 1 hr at 100nM (not depicted) or 1000 nM. Subsequently, 5×10⁴ Jurkat-Vδ1 were added to each well. After 24 hr Jurkat-Vδ1 cells were harvested and analysed for CD69 expression by flow cytometry. As can be seen in figure 2B, addition of 1D12 during the co-culture completely abrogated activation of Vδ1-Jurkat, whereas 1D22 had only a limited impact on expression of the activation marker CD69. Incubation with 100 nM or 1000 nM yielded similar results (data not shown).

To evaluate the effect of 1D12 and 1D22 on CD1d-tetramer binding on Jurkat-Vδ1 cells, endogenous or sulfatide-loaded CD1d-PE tetramers were incubated with either PBS (control), 1D12 or 1D22 (ratio VHH:C01d of ~4:1) for 30 min at room temperature after which tetramers were added to Jurkat-Vδ1 cells (final concentration tetramer 2 µg/ml) in combination with CD3-APC and incubated for 45 min at 4 degrees Celsius. Data was analyzed by flow cytometry. Incubation of sulfatide-loaded COld tetramers with 1D12 prevented binding to Jurkat-Vδ1 cells completely, while 1D22 had only a limited impact (figure 2A). These data support the ability of the anti-CD1d VHH to modulate reactivity of specific CDld restricted T-cells, which contrast sharply with known mAb, such as 51.1 mAb, that block CD1d-TCR interaction of a broad range of CD1d-restricted T-cells (Nambiar et al. (2015) MAbs 7:638; Migalovich Sheikhet et al. (2018) Front Immunol 9:753).

### Example 3

### Dual activation of iNKT and Vγ9Vδ2-T cells by a bispecific anti-CD1d-anti-Vγ9Vδ2 TCR VHH

Previously, well characterized anti-Vγ9Vδ2-TCR VHH have been fused to VHHs specific for tumor-associated antigens for anti-tumor therapeutic purposes. CDld is expressed on various (hematological) malignancies and on tumor associated macrophages and myeloid-derived suppressor cells and could therefore be used as an anti-cancer therapeutic target. To evaluate the ability of 1D12-5C8 to induce dual activation of iNKT and Vγ9Vδ2-T-cells resulting in tumor target lysis, 1×10⁵ CCRF-CEM cells were incubated with either 5×10⁴ iNKT cells, 5×10⁴ Vγ9Vδ2-T cells or 5×10⁴ mixed iNKT/Vγ9Vδ2-T (1:1 ratio) in the presence of medium alone, monovalent 1D12 or bispecific 1D12-5C8. After 4 hr degranulation of effector cells was measured by CD107a expression and analyzed by flow cytometry. To asses cytotoxicity towards target cells, living CCRF-CEM cells (Annexin V and 7-AAD negative) were quantified after 16h co-culture using flow cytometry cell counting beads.

To determine the capacity of 1D12-5C8 to support iNKT and Vγ9Vδ2-T expansion and control tumor growth, freshly isolated iNKT and Vγ9Vδ2-T from the same donor were expanded for 1 week. 5×10⁴ MM.1s-CD1d cells were subsequently incubated with medium or 1D12-5C8 (50 nM) for 30 min after which iNKT, Vγ9Vδ2-T cells or a mixture thereof (2:3 ratio) was added in a 10:1 target:effector ratio. Living MM.1s-CD1d (or MOLM-13 or NOMO-1), iNKT and Vγ9Vδ2-T cells (7-AAD negative) were quantified after 7 days using flow cytometry cell counting beads.

As can be seen in figure 3a, robust simultaneous degranulation of iNKT and Vγ9Vδ2-T cells was only observed in the presence of 1D12-5C8. Moreover, effector cell activation resulted in a striking reduction in living tumor cells (fig. 3b).

The unfavourable effector to target ratio *in vivo* usually requires expansion of tumor targeting effector cells to control tumor growth. To investigate whether the bispecific 1D12-5C8 VHH could induce both effector cell expansion and tumor control in such a setting, MM.1s-CD1d cells were incubated with 1D12-5C8, after which iNKT, Vγ9Vδ2-T cells or a mixture thereof were added at an effector to target ratio of 1:10. The ability of 1D12-5C8 to induce expansion and control tumor growth was evaluated after a 7 day co-culture by flow cytometric quantification of these cells. As can be seen in figure 4a expansion of iNKT was observed in the presence of the bispecific construct. However, Vγ9Vδ2-T cells only showed expansion in the presence of both iNKT cells and the bispecific construct. Moreover, robust tumor growth control was induced by the bispecific construct in combination with effector cells (fig. 4b). Similar, tumor growth control and effector cell expansion were observed with the acute myeloid leukemia tumor cell lines MOLM-13 and NOMO-1, underscoring the powerful anti-tumor efficacy and broad applicability of this bispecific anti-CD1d-anti-Vγ9Vδ2-TCR VHH.

### Example 4

### Binding competition of 1D12 and 1D12-5C8

To evaluate whether 1D12 binding would interfere with 1D12-5C8 binding, 1×10⁵ MM1s-CDld cells were incubated with either PBS (negative control, NC), 1D12 (1000 nM), 1D22 (1000 nM) or anti-CD1d mAb 51.1 (100 nM) for 45 min after which PBS (NC) or NHS-biotin (ThermoFischer Scientific Inc., Waltham, MA) linked 1D12-5C8 (100 nM) was added for an additional 30 min at 4 degrees Celsius. After extensive washing samples were stained with streptavidin-APC (eBioscience, San Diego, CA) and analyzed by flow cytometry.

To evaluate competition between 1D12 and 1D12-5C8, CDld expressing MM cells were sequentially incubated with either PBS, 1D12, 1D22 (which should not interfere with 1D12-5C8 binding) or anti-CD1d mAb 51.1) followed by biotinylated 1D12-5C8. The ability of 1D12-5C8 to complex with CDld was then determined by analysing binding of streptavidin-APC by flow cytometry. As can be seen in figure 5, pre-incubation of 1D12 or anti-CD1d mAb 51.1, but not 1D22, greatly reduced 1D12-5C8 binding.

### Example 5

### In vivo xenograft mouse multiple myeloma (MM) model

The anti-tumour efficacy of bispecific CD1d/Vδ2 binding antibody 1D12-5C8 was studied in an *in vivo* model where mice were i.v. inoculated with MM.1s.mCherry/luc.C01d cells to establish a disseminated MM model followed by three i.v. infusions of human iNKT cells, human γδ T cells or a mixture thereof starting 7 days post tumour inoculation whether or not in combination with 1D12-5C8. Whereas biweekly i.p. dosing of 1D12-5C8 alone had no effect (median survival 47 days versus 49.5 days, P>0,05), combination treatment of 1D12-5C8 and iNKT cells significantly (p<0.0001) prolonged survival compared to iNKT alone (median survival 58.5 days) with all mice being alive at termination of the study (day 90). Compared to treatment with human γδ T cells only, treatment of human γδ T cells and 1D12-5C8 showed a trend towards increased median survival from 48 days to 60 days (p=0.16). Infusion of both iNKT cells and γδ T cells with biweekly i.p. dosing 1D12-5C8 significantly prolonged survival with 7/8 mice being alive at study termination (day 90) (p>0.0001) compared to the mixture of the cells alone without antibody (median survival 55 days).

## Claims

1. Binding molecule comprising a first binding moiety of an antibody that is able to compete with the antibody set forth in SEQ ID NO:4 in binding to a CDld molecule and comprising a second binding moiety of an antibody that is able to specifically bind to a Vγ9Vδ2-TCR, wherein the binding molecule is able to activate iNKT cells and wherein the binding molecule is able to activate Vγ9Vδ2 T cells.

2. Binding molecule according to claim 1, wherein the binding molecule is able to reduce Vδ1 T cell activation.

3. Binding molecule according to any one of the preceding claims, wherein the first and/or second binding moiety is a single domain antibody.

4. Binding molecule according to any one of the preceding claims, wherein the first binding moiety comprises a CDR1 sequence according to SEQ ID NO: 1, a CDR2 sequence according to SEQ ID NO: 2, and/or a CDR3 sequence according to SEQ ID NO: 3, or any of those sequences wherein, independently, any of the amino acids, preferably at most 4 amino acids, more preferably at most 3, more preferably at most 2, most preferably at most 1, has been conservatively substituted, according to the following table:
| Residue | Conservative Substitutions | Residue | Conservative Substitutions |
|---|---|---|---|
| Ala | Ser | Leu | Ile; Val |
| Arg | Lys | Lys | Arg; Gin |
| Asn | Gln; His | Met | Leu; Ile |
| Asp | Glu | Phe | Met; Leu; Tyr |
| Gln | Asn | Ser | Thr, Gly |
| Cys | Ser | Thr | Ser; Val |
| Glu | Asp | Trp | Tyr |
| Gly | Pro | Tyr | Trp; Phe |
| His | Asn; Gin | Val | Ile; Leu |
| Ile | Leu, Val | | |

5. Binding molecule according to any one of the preceding claims, wherein the first binding moiety comprises the sequence set forth in SEQ ID NO: 85.

6. Binding molecule according to any one of the preceding claims, wherein the second binding moiety comprises a CDR 1 sequence according SEQ ID NO: 5, a CDR 2 sequence according to SEQ ID NO: 6 and/or a CDR 3 sequence according to SEQ ID NO: 7; or a CDR 1 sequence according SEQ ID NO: 8, a CDR 2 sequence according to SEQ ID NO: 9 and/or a CDR 3 sequence according to SEQ ID NO: 10; or a CDR 1 sequence according SEQ ID NO: 11, a CDR 2 sequence according to SEQ ID NO: 12 and/or a CDR 3 sequence according to SEQ ID NO: 13; or a CDR 1 sequence according SEQ ID NO: 14, a CDR 2 sequence according to SEQ ID NO: 15 and/or a CDR 3 sequence according to SEQ ID NO: 16; or a CDR 1 sequence according SEQ ID NO: 17, a CDR 2 sequence according to SEQ ID NO: 18 and/or a CDR 3 sequence according to SEQ ID NO: 19; or a CDR 1 sequence according SEQ ID NO: 20, a CDR 2 sequence according to SEQ ID NO: 21 and/or a CDR 3 sequence according to SEQ ID NO: 22; or a CDR 1 sequence according SEQ ID NO: 23, a CDR 2 sequence according to SEQ ID NO: 24 and/or a CDR 3 sequence according to SEQ ID NO: 25; or a CDR 1 sequence according SEQ ID NO: 26, a CDR 2 sequence according to SEQ ID NO: 27 and/or a CDR 3 sequence according to SEQ ID NO: 28; or a CDR 1 sequence according SEQ ID NO: 29, a CDR 2 sequence according to SEQ ID NO: 30 and/or a CDR 3 sequence according to SEQ ID NO: 31; or a CDR 1 sequence according SEQ ID NO: 32, a CDR 2 sequence according to SEQ ID NO: 33 and/or a CDR 3 sequence according to SEQ ID NO: 34; or a CDR 1 sequence according SEQ ID NO: 35, a CDR 2 sequence according to SEQ ID NO: 36 and/or a CDR 3 sequence according to SEQ ID NO: 37; or a CDR 1 sequence according SEQ ID NO: 38, a CDR 2 sequence according to SEQ ID NO: 39 and/or a CDR 3 sequence according to SEQ ID NO: 40; or a CDR 1 sequence according SEQ ID NO: 41, a CDR 2 sequence according to SEQ ID NO: 42 and/or a CDR 3 sequence according to SEQ ID NO: 43; or a CDR 1 sequence according SEQ ID NO: 44, a CDR 2 sequence according to SEQ ID NO: 45 and/or a CDR 3 sequence according to SEQ ID NO: 46; or a CDR 1 sequence according SEQ ID NO: 47, a CDR 2 sequence according to SEQ ID NO: 48 and/or a CDR 3 sequence according to SEQ ID NO: 49; or a CDR 1 sequence according SEQ ID NO: 50, a CDR 2 sequence according to SEQ ID NO: 51 and/or a CDR 3 sequence according to SEQ ID NO: 52; or a CDR 1 sequence according SEQ ID NO: 53, a CDR 2 sequence according to SEQ ID NO: 54 and/or a CDR 3 sequence according to SEQ ID NO: 55; or a CDR 1 sequence according SEQ ID NO: 56, a CDR 2 sequence according to SEQ ID NO: 57 and/or a CDR 3 sequence according to SEQ ID NO: 58, or any of those sequences wherein, independently, any of the amino acids, preferably at most 4 amino acids, more preferably at most 3, more preferably at most 2, most preferably at most 1, has been conservatively substituted, according to the following table:
| Residue | Conservative Substitutions | Residue | Conservative Substitutions |
|---|---|---|---|
| Ala | Ser | Leu | Ile; Val |
| Arg | Lys | Lys | Arg; Gin |
| Asn | Gln; His | Met | Leu; Ile |
| Asp | Glu | Phe | Met; Leu; Tyr |
| Gln | Asn | Ser | Thr, Gly |
| Cys | Ser | Thr | Ser; Val |
| Glu | Asp | Trp | Tyr |
| Gly | Pro | Tyr | Trp; Phe |
| His | Asn; Gin | Val | Ile; Leu |
| Ile | Leu, Val | | |

7. Binding molecule according to any one of the preceding claims, wherein the second binding moiety comprises the sequence set forth in SEQ ID NO: 86 or SEQ ID NO: 88.

8. Binding molecule according to any one of the preceding claims, wherein the binding molecule comprises the sequence set forth in SEQ ID NO: 87.

9. Binding molecule according to any one of the preceding claims, further comprising a tumor targeting moiety.

10. Binding molecule according to any one of the preceding claims for use as a medicament.

11. Binding molecule according to any one of the preceding claims for use in the treatment of a tumor.

12. Binding molecule for use according to 11, wherein the tumor is selected from hematological malignancies such as T cell lymphoma, multiple myeloma, acute myeloid leukemia, acute lymphoblastic leukemia, chronic lymphocytic leukemia, mantle cell lymphoma, B cell lymphoma, smoldering myeloma, Hodgkin lymphoma, myelomonocytic leukemias, lymphoplasmacytic lymphoma, hairy cell leukemia, and splenic marginal zone lymphoma, or solid tumors, such as renal cell carcinoma, melanoma, colorectal carcinoma, head and neck cancer, breast cancer, prostate cancer, lung cancer, pancreatic cancer, gastro-esophageal cancer, small bowel carcinoma, central nervous system tumors, medulloblastomas, hepatocellular carcinoma, ovarian cancer, glioma, neuroblastoma, urothelial carcinomas, bladder cancer, sarcoma, penile cancer, basal cell carcinoma, merkel cell carcinoma, neuroendocrine carcinoma, neuroendocrine tumors, carcinoma of unknown primary (CUP), thymoma, vulvar cancer, cervical carcinoma, testicular cancer, cholangiocarcinoma, appendicular carcinoma, mesothelioma, ampullary carcinoma, anal cancer, and choriocarcinoma.

13. Pharmaceutical composition comprising a binding molecule according to claim 1 and a pharmaceutically acceptable excipient.

## Patentansprüche

1. Bindungsmolekül, das einen ersten bindenden Rest eines Antikörpers umfasst, der dazu fähig ist, mit dem in SEQ ID Nr.: 4 aufgeführten Antikörper bezüglich einer Bindung an einem CD1d-Molekül zu konkurrieren und das einen zweiten bindenden Rest eines Antikörpers umfasst, der dazu fähig ist, an Vγ9Vδ2-TCR spezifisch zu binden, wobei das Bindungsmolekül dazu fähig ist, iNKT-Zellen zu aktivieren, und wobei das Bindungsmolekül dazu fähig ist, Vγ9Vδ2-T-Zellen zu aktivieren.

2. Bindungsmolekül nach Anspruch 1, wobei das Bindungsmolekül dazu fähig ist, eine Aktivierung von Vδ1-T-Zellen zu reduzieren.

3. Bindungsmolekül nach einem der vorhergehenden Ansprüche, wobei der erste und/oder der zweite bindende Rest ein Einzeldomänenantikörper sind.

4. Bindungsmolekül nach einem der vorhergehenden Ansprüche, wobei der erste bindende Rest eine CDR1-Sequenz gemäß SEQ ID Nr.: 1, eine CDR2-Sequenz gemäß SEQ ID Nr.: 2 und/oder eine CDR3-Sequenz gemäß SEQ ID Nr.: 3 oder eine beliebige dieser Sequenzen umfasst, wobei unabhängig jede der Aminosäuren, vorzugsweise höchstens 4, stärker bevorzugt höchstens 3, stärker bevorzugt höchstens 2 Aminosäuren, am meisten bevorzugt höchstens 1 Aminosäure, gemäß der folgenden Tabelle konservativ substituiert worden ist:
| Rest | Konservative Substitutionen | Rest | Konservative Substitutionen |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr, Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

5. Bindungsmolekül nach einem der vorhergehenden Ansprüche, wobei der erste bindende Rest die in SEQ ID Nr.: 85 aufgeführte Sequenz umfasst.

6. Bindungsmolekül nach einem der vorhergehenden Ansprüche, wobei der zweite bindende Rest eine CDR-1-Sequenz gemäß SEQ ID Nr.: 5, eine CDR-2-Sequenz gemäß SEQ ID Nr.: 6 und/oder eine CDR-3-Sequenz gemäß SEQ ID Nr.: 7 oder eine CDR-1-Sequenz gemäß SEQ ID Nr.: 8, eine CDR-2-Sequenz gemäß SEQ ID Nr.: 9 und/oder eine CDR-3-Sequenz gemäß SEQ ID Nr.: 10 oder eine CDR-1-Sequenz gemäß SEQ ID Nr.: 11, eine CDR-2-Sequenz gemäß SEQ ID Nr.: 12 und/oder eine CDR-3-Sequenz gemäß SEQ ID Nr.: 13 oder eine CDR-1-Sequenz gemäß SEQ ID Nr.: 14, eine CDR-2-Sequenz gemäß SEQ ID Nr.: 15 und/oder eine CDR-3-Sequenz gemäß SEQ ID Nr.: 16 oder eine CDR-1-Sequenz gemäß SEQ ID Nr.: 17, eine CDR-2-Sequenz gemäß SEQ ID Nr.: 18 und/oder eine CDR-3-Sequenz gemäß SEQ ID Nr.: 19 oder eine CDR-1-Sequenz gemäß SEQ ID Nr.: 20, eine CDR-2-Sequenz gemäß SEQ ID Nr.: 21 und/oder eine CDR-3-Sequenz gemäß SEQ ID Nr.: 22 oder eine CDR-1-Sequenz gemäß SEQ ID Nr.: 23, eine CDR-2-Sequenz gemäß SEQ ID Nr.: 24 und/oder eine CDR-3-Sequenz gemäß SEQ ID Nr.: 25 oder eine CDR-1-Sequenz gemäß SEQ ID Nr.: 26, eine CDR-2-Sequenz gemäß SEQ ID Nr.: 27 und/oder eine CDR-3-Sequenz gemäß SEQ ID Nr.: 28 oder eine CDR-1-Sequenz gemäß SEQ ID Nr.: 29, eine CDR-2-Sequenz gemäß SEQ ID Nr.: 30 und/oder eine CDR-3-Sequenz gemäß SEQ ID Nr.: 31 oder eine CDR-1-Sequenz gemäß SEQ ID Nr.: 32, eine CDR-2-Sequenz gemäß SEQ ID Nr.: 33 und/oder eine CDR-3-Sequenz gemäß SEQ ID Nr.: 34 oder eine CDR-1-Sequenz gemäß SEQ ID Nr.: 35, eine CDR-2-Sequenz gemäß SEQ ID Nr.: 36 und/oder eine CDR-3-Sequenz gemäß SEQ ID Nr.: 37 oder eine CDR-1-Sequenz gemäß SEQ ID Nr.: 38, eine CDR-2-Sequenz gemäß SEQ ID Nr.: 39 und/oder eine CDR-3-Sequenz gemäß SEQ ID Nr.: 40 oder eine CDR-1-Sequenz gemäß SEQ ID Nr.: 41, eine CDR-2-Sequenz gemäß SEQ ID Nr.: 42 und/oder eine CDR-3-Sequenz gemäß SEQ ID Nr.: 43 oder eine CDR-1-Sequenz gemäß SEQ ID Nr.: 44, eine CDR-2-Sequenz gemäß SEQ ID Nr.: 45 und/oder eine CDR-3-Sequenz gemäß SEQ ID Nr.: 46 oder eine CDR-1-Sequenz gemäß SEQ ID Nr.: 47, eine CDR-2-Sequenz gemäß SEQ ID Nr.: 48 und/oder eine CDR-3-Sequenz gemäß SEQ ID Nr.: 49 oder eine CDR-1-Sequenz gemäß SEQ ID Nr.: 50, eine CDR-2-Sequenz gemäß SEQ ID Nr.: 51 und/oder eine CDR-3-Sequenz gemäß SEQ ID Nr.: 52 oder eine CDR-1-Sequenz gemäß SEQ ID Nr.: 53, eine CDR-2-Sequenz gemäß SEQ ID Nr.: 54 und/oder eine CDR-3-Sequenz gemäß SEQ ID Nr.: 55 oder eine CDR-1-Sequenz gemäß SEQ ID Nr.: 56, eine CDR-2-Sequenz gemäß SEQ ID Nr.: 57 und/oder eine CDR-3-Sequenz gemäß SEQ ID Nr.: 58 oder eine beliebige dieser Sequenzen umfasst, wobei unabhängig jede der Aminosäuren, vorzugsweise höchstens 4, stärker bevorzugt höchstens 3, stärker bevorzugt höchstens 2 Aminosäuren, am meisten bevorzugt höchstens 1 Aminosäure, gemäß der folgenden Tabelle konservativ substituiert worden ist:
| Rest | Konservative Substitutionen | Rest | Konservative Substitutionen |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr, Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

7. Bindungsmolekül nach einem der vorhergehenden Ansprüche, wobei der zweite bindende Rest die in SEQ ID Nr.: 86 oder SEQ ID Nr.: 88 aufgeführte Sequenz umfasst.

8. Bindungsmolekül nach einem der vorhergehenden Ansprüche, wobei das Bindungsmolekül die in SEQ ID Nr.: 87 aufgeführte Sequenz umfasst.

9. Bindungsmolekül nach einem der vorhergehenden Ansprüche, das weiterhin einen Tumortargeting-Rest umfasst.

10. Bindungsmolekül nach einem der vorhergehenden Ansprüche zur Verwendung als Medikament.

11. Bindungsmolekül nach einem der vorhergehenden Ansprüche zur Verwendung bei der Behandlung eines Tumors.

12. Bindungsmolekül zur Verwendung nach 11, wobei der Tumor ausgewählt ist aus hämatologischen bösartigen Erkrankungen, wie einem T-Zell-Lymphom, multiplen Myelom, akuter myeloischer Leukämie, akuter lymphoblastischer Leukämie, chronischer lymphozytischer Leukämie, Mantelzell-Lymphom, B-Zell-Lymphom, schwelenden Myelom, Hodgkin-Lymphom, myelomonozytären Leukämien, lymphoplasmazytären Lymphom, Haarzell-Leukämie und einem Marginalzonen-Lymphom der Milz, oder soliden Tumoren, wie einem Nierenzellkarzinom, Melanom, Kolorektalkarzinom, Kopf- und Halskrebs, Brustkrebs, Prostatakrebs, Lungenkrebs, Pankreaskrebs, gastroösophagealen Karzinom, Dünndarmkarzinom, Tumoren des Zentralnervensystems, Medulloblastomen, hepatozellulären Karzinom, Ovarialkrebs, Gliom, Neuroblastom, Urothelkarzinomen, Blasenkrebs, Sarkom, Peniskrebs, Basalzellkarzinom, Merkelzell-Karzinom, neuroendokrinen Karzinom, neuroendokrinen Tumoren, Karzinom mit unbekannter Primärlokalisation (CUP), Thymom, Vulvakrebs, Zervixkarzinom, Hodenkrebs, Cholangiokarzinom, Appendixkarzinom, Mesotheliom, ampullären Karzinom, Analkarzinom und Choriokarzinom.

13. Pharmazeutische Zusammensetzung, die ein Bindungsmolekül nach Anspruch 1 und einen pharmazeutisch annehmbaren Hilfsstoff umfasst.

## Revendications

1. Molécule de liaison comprenant une première fraction de liaison d'un anticorps qui est capable d'entrer en compétition avec l'anticorps indiqué dans la SEQ ID NO : 4 dans la liaison à une molécule CD1d et comprenant une deuxième fraction de liaison d'un anticorps qui est capable de se lier spécifiquement à un Vγ9Vδ2-TCR, la molécule de liaison étant capable d'activer des cellules iNKT et la molécule de liaison étant capable d'activer des cellules T Vγ9Vδ2.

2. Molécule de liaison selon la revendication 1, la molécule de liaison étant capable de réduire l'activation de cellules T Vδ1.

3. Molécule de liaison selon l'une quelconque des revendications précédentes, la première et/ou deuxième fraction de liaison étant un anticorps à domaine unique.

4. Molécule de liaison selon l'une quelconque des revendications précédentes, la première fraction de liaison comprenant une séquence CDR1 selon la SEQ ID NO : 1, une séquence CDR2 selon la SEQ ID NO : 2, et/ou une séquence CDR3 selon la SEQ ID NO : 3, ou l'une quelconque de ces séquences dans lesquelles, indépendamment, l'un quelconque des acides aminés, préférablement au plus 4 acides aminés, plus préférablement au plus 3, plus préférablement au plus 2, le plus préférablement au plus 1, a été substitué de manière conservatrice, selon le tableau suivant :
| Résidu | Substitutions conservatrices | Résidu | Substitutions conservatrices |
|---|---|---|---|
| Ala | Ser | Leu | Ile ; Val |
| Arg | Lys | Lys | Arg ; Gln |
| Asn | Gln ; His | Met | Leu ; Ile |
| Asp | Glu | Phe | Met ; Leu ; Tyr |
| Gln | Asn | Ser | Thr, Gly |
| Cys | Ser | Thr | Ser ; Val |
| Glu | Asp | Trp | Tyr |
| Gly | Pro | Tyr | Trp ; Phe |
| His | Asn ; Gln | Val | Ile ; Leu |
| Ile | Leu, Val | | |

5. Molécule de liaison selon l'une quelconque des revendications précédentes, la première fraction de liaison comprenant la séquence indiquée dans la SEQ ID NO : 85.

6. Molécule de liaison selon l'une quelconque des revendications précédentes, la deuxième fraction de liaison comprenant une séquence CDR 1 selon la SEQ ID NO : 5, une séquence CDR 2 selon la SEQ ID NO : 6, et/ou une séquence CDR 3 selon la SEQ ID NO : 7 ; ou une séquence CDR 1 selon la SEQ ID NO : 8, une séquence CDR 2 selon la SEQ ID NO : 9, et/ou une séquence CDR 3 selon la SEQ ID NO : 10 ; ou une séquence CDR 1 selon la SEQ ID NO : 11, une séquence CDR 2 selon la SEQ ID NO : 12, et/ou une séquence CDR 3 selon la SEQ ID NO : 13 ; ou une séquence CDR 1 selon la SEQ ID NO : 14, une séquence CDR 2 selon la SEQ ID NO : 15, et/ou une séquence CDR 3 selon la SEQ ID NO : 16 ; ou une séquence CDR 1 selon la SEQ ID NO : 17, une séquence CDR 2 selon la SEQ ID NO : 18, et/ou une séquence CDR 3 selon la SEQ ID NO : 19 ; ou une séquence CDR 1 selon la SEQ ID NO : 20, une séquence CDR 2 selon la SEQ ID NO : 21, et/ou une séquence CDR 3 selon la SEQ ID NO : 22 ; ou une séquence CDR 1 selon la SEQ ID NO : 23, une séquence CDR 2 selon la SEQ ID NO : 24, et/ou une séquence CDR 3 selon la SEQ ID NO : 25 ; ou une séquence CDR 1 selon la SEQ ID NO : 26, une séquence CDR 2 selon la SEQ ID NO : 27, et/ou une séquence CDR 3 selon la SEQ ID NO : 28 ; ou une séquence CDR 1 selon la SEQ ID NO : 29, une séquence CDR 2 selon la SEQ ID NO : 30, et/ou une séquence CDR 3 selon la SEQ ID NO : 31 ; ou une séquence CDR 1 selon la SEQ ID NO : 32, une séquence CDR 2 selon la SEQ ID NO : 33, et/ou une séquence CDR 3 selon la SEQ ID NO : 34 ; ou une séquence CDR 1 selon la SEQ ID NO : 35, une séquence CDR 2 selon la SEQ ID NO : 36, et/ou une séquence CDR 3 selon la SEQ ID NO : 37 ; ou une séquence CDR 1 selon la SEQ ID NO : 38, une séquence CDR 2 selon la SEQ ID NO : 39, et/ou une séquence CDR 3 selon la SEQ ID NO : 40 ; ou une séquence CDR 1 selon la SEQ ID NO : 41, une séquence CDR 2 selon la SEQ ID NO : 42, et/ou une séquence CDR 3 selon la SEQ ID NO : 43 ; ou une séquence CDR 1 selon la SEQ ID NO : 44, une séquence CDR 2 selon la SEQ ID NO : 45, et/ou une séquence CDR 3 selon la SEQ ID NO : 46 ; ou une séquence CDR 1 selon la SEQ ID NO : 47, une séquence CDR 2 selon la SEQ ID NO : 48, et/ou une séquence CDR 3 selon la SEQ ID NO : 49 ; ou une séquence CDR 1 selon la SEQ ID NO : 50, une séquence CDR 2 selon la SEQ ID NO : 51, et/ou une séquence CDR 3 selon la SEQ ID NO : 52 ; ou une séquence CDR 1 selon la SEQ ID NO : 53, une séquence CDR 2 selon la SEQ ID NO : 54, et/ou une séquence CDR 3 selon la SEQ ID NO : 55 ; ou une séquence CDR 1 selon la SEQ ID NO : 56, une séquence CDR 2 selon la SEQ ID NO : 57, et/ou une séquence CDR 3 selon la SEQ ID NO : 58, ou l'une quelconque de ces séquences dans lesquelles, indépendamment, l'un quelconque des acides aminés, préférablement au plus 4 acides aminés, plus préférablement au plus 3, plus préférablement au plus 2, le plus préférablement au plus 1, a été substitué de manière conservatrice, selon le tableau suivant :
| Résidu | Substitutions conservatrices | Résidu | Substitutions conservatrices |
|---|---|---|---|
| Ala | Ser | Leu | Ile ; Val |
| Arg | Lys | Lys | Arg ; Gln |
| Asn | Gln ; His | Met | Leu ; Ile |
| Asp | Glu | Phe | Met ; Leu ; Tyr |
| Gln | Asn | Ser | Thr, Gly |
| Cys | Ser | Thr | Ser ; Val |
| Glu | Asp | Trp | Tyr |
| Gly | Pro | Tyr | Trp ; Phe |
| His | Asn ; Gln | Val | Ile ; Leu |
| Ile | Leu, Val | | |

7. Molécule de liaison selon l'une quelconque des revendications précédentes, la deuxième fraction de liaison comprenant la séquence indiquée dans la SEQ ID NO : 86 ou la SEQ ID NO : 88.

8. Molécule de liaison selon l'une quelconque des revendications précédentes, la molécule de liaison comprenant la séquence indiquée dans la SEQ ID NO : 87.

9. Molécule de liaison selon l'une quelconque des revendications précédentes, comprenant en outre une fraction de ciblage de tumeur.

10. Molécule de liaison selon l'une quelconque des revendications précédentes pour une utilisation en tant que médicament.

11. Molécule de liaison selon l'une quelconque des revendications précédentes pour une utilisation dans le traitement d'une tumeur.

12. Molécule de liaison pour une utilisation selon la revendication 11, la tumeur étant choisie parmi les hémopathies malignes telles que le lymphome à cellules T, le myélome multiple, la leucémie myéloïde aiguë, la leucémie lymphoblastique aiguë, la leucémie lymphoïde chronique, le lymphome à cellules du manteau, le lymphome à cellules B, le myélome latent, le lymphome de Hodgkin, les leucémies myélomonocytaires, le lymphome lymphoplasmocytaire, la leucémie à tricholeucocytes et le lymphome de la zone marginale splénique, ou les tumeurs solides, telles que le carcinome à cellules rénales, le mélanome, le carcinome colorectal, le cancer de la tête et du cou, le cancer du sein, le cancer de la prostate, le cancer du poumon, le cancer du pancréas, le cancer gastro-œsophagien, le carcinome de l'intestin grêle, les tumeurs du système nerveux central, les médulloblastomes, le carcinome hépatocellulaire, le cancer de l'ovaire, le gliome, le neuroblastome, les carcinomes urothéliaux, le cancer de la vessie, le sarcome, le cancer du pénis, le carcinome basocellulaire, le carcinome à cellules de Merkel, le carcinome neuroendocrinien, les tumeurs neuroendocrines, le carcinome primitif inconnu (CUP), le thymome, le cancer de la vulve, le carcinome du col de l'utérus, le cancer des testicules, le cholangiocarcinome, le carcinome appendiculaire, le mésothéliome, le carcinome ampullaire, le cancer anal et le choriocarcinome.

13. Composition pharmaceutique comprenant une molécule de liaison selon la revendication 1 et un excipient pharmaceutiquement acceptable.
